# EUROPEAN PATENT APPLICATION

(11) **EP 2 554 660 A1**
(43) Date of publication of application: **06.02.2013**
(21) Application number: 11762825.5
(22) Date of filing: 29.03.2011
(51) Int. Cl.: C12N 5/077, A61K 35/28, A61K 38/00, A61P 19/00, C12N 5/0775

(54) **INTERVERTEBRAL DISC NUCLEUS PULPOSUS STEM/PROGENITOR CELL, METHOD FOR CULTURING SAME, AND APPLICATION**

(30) Priority: 30.03.2010 JP 2010077743
(71) Applicant: Tokai University Educational System, Tokyo 151-0063 (JP)
(72) Inventor: SAKAI, Daisuke, Isehara-shi Kanagawa 259-1193 (JP); MOCHIDA, Joji, Isehara-shi Kanagawa 259-1193 (JP); ANDO, Kiyoshi, Isehara-shi Kanagawa 259-1193 (JP); NAKAMURA, Yoshihiko, Isehara-shi Kanagawa 259-1193 (JP)
(74) Representative: Leathley, Anna Elisabeth
(86) International application number: PCT/JP2011/057750
(87) International publication number: WO 2011/122601

(57) **Abstract**

The purpose of the present invention is to provide intervertebral disk nucleus pulposus stem cells or progenitor cells that may be used for treatment of intervertebral disk disorders. The present invention provides an intervertebral disk nucleus pulposus cell characterized by being isolated from the intervertebral disk nucleus pulposus of a vertebrate and being positive for at least one surface marker from among Tie2 and GD2. That is, the present invention provides an intervertebral disk nucleus pulposus stem cell characterized by being at least Tie2-positive for the surface marker and possessing self-renewal ability as well as multipotency capable of differentiating into adipocytes, osteocytes, chondrocytes and neurons. The present invention also provides an intervertebral disk nucleus pulposus progenitor cell characterized by being at least Tie2-negative and GD2-positive for the surface marker and capable of differentiating into any of adipocytes, osteocytes, chondrocytes and neurons.

## Description

### TECHNICAL FIELD

The present invention relates to a stem cell and a progenitor cell included in the nucleus pulposus of an intervertebral disk, and more specifically, relates to a marker specifying said cells. Moreover, the present invention also relates to the cultivation method of said stem cell and progenitor cell, and the use thereof.

### BACKGROUND ART

Lower back pain is a common malady rated second among persons with subjective symptoms¹ and experienced by two thirds of adults at least once, making it a social problem in terms of work impairment and medical economics. Intervertebral disk disorder, which is said to cause 20% of lower back pain cases³, causes an unbalanced movable site (motion segment) and is a major problem, possibly inducing intervertebral disk herniation, spinal osteophytosis, spinal canal stenosis, spondylolisthesis, etc. Intervertebral disk disorder, which is pathologically referred to as intervertebral disk degeneration, is an irreversible change with no clinically effective therapy currently in existence, so the development of a new therapy is under study at many domestic and overseas research institutes. In the future, understanding the intervertebral disk at the cellular/molecular level will be essential in developing a new therapy on a scientific basis; however, the onset, component cell differentiation, homeostasis, and degeneration mechanism of intervertebral disks remain unknown in comparison with bones and articular cartilages^{4, 5}.

The intervertebral disk is a donut-shaped cartilaginous organ shaped from a nucleus pulposus (NP) in the center, an annulus fibrosus (AF), which is a fibrous cartilage encircling the surrounding region thereof many times, and a cartilaginous endplate (EP) connecting adjacent vertebras from top to bottom. The gelatin-like NP is a avascular organ and contains a high proportion of an extracellular matrix (ECM) configured from a large proteoglycan and collagen. The NP is embryologically derived from notochord; however, it is known that the surrounding organs are generated from mesenchyme. Specific cell markers or the like of nucleus pulposus cells have been unknown to date, so there was no other choice but to rely on morphological characteristics alone when distinguishing from other cells, making a detailed analysis impossible. It has been reported that notochord-derived nucleus pulposus cells disappear early in the lifetime of some vertebrates, including those in humans. Following the disappearance of notochord-derived nucleus pulposus cells, a chondroid cell, the origin of which has not yet been definede but morphologically similar to a chondrocyte, shapes the nucleus pulposus. This cellular phenotype conversion affects the ECM composition and is believed to be involved in subsequent aging and degeneration of the intervertebral disk such as by decreased water content, tissue fibrosis, etc., becoming largely involved in lower back pain and degenerative lumbar spine disease later on. Meanwhile, considering that intervertebral disk degeneration is rarely observed in many animals including mice, rats, pigs, etc. that maintain the notochord-derived nucleus pulposus cells throughout their lifetime, it is believed that clarifying the control mechanism of notochord-derived nucleus pulposus cells and other nucleus pulposus cells will provide useful information in the prevention of intervertebral disk disorders and regeneration of intervertebral disks^{6, 7}.

### CITATION LIST

### Non-Patent Literatures

Non-Patent Literature 1: Deyo, R.A. & Weinstein, J.N. Low Back Pain. N Engl J Med. 344, 363-70 (2001).
Non-Patent Literature 2: Carragee, E. Surgical treatment of lumbar disk disorders. JAMA. 296, 2485-7 (2006).
Non-Patent Literature 3: Antoniou, J. et al. The human lumbar intervertebral disc: evidence for changes in the biosynthesis and denaturation of the extracellular matrix with growth, maturation, ageing, and degeneration. J Clin Invest. 98, 996-1003 (1996).
Non-Patent Literature 4: Seki, S. et al. A functional SNP in CILP, encoding cartilage intermediate layer protein, is associated with susceptibility to lumbar disc disease. Nat Genet. 37, 607-12 (2005).
Non-Patent Literature 5: Annunen, S. et al. An allele of COL9A2 associated with intervertebral disc disease. Science. 285, 409-12 (1999).
Non-Patent Literature 6: Guehring, T. et al. Notochordal intervertebral disc cells: sensitivity to nutrient deprivation. Arthritis Rheum. 60, 1026-34 (2009).
Non-Patent Literature 7: Yang, F., Leung, V.Y., Luk. K.D., Chan, D. & Cheung, K.M. Injury-induced sequential transformation of notochordal nucleus pulposus to chondrogenic and fibrocartilaginous phenotype in the mouse. J Pathol. 218, 113-21 (2009).
Non-Patent Literature 8: Risbud, M.V. et al. Evidence for skeletal progenitor cells in the degenerate human intervertebral disc. Spine. 32, 2537-44 (2007).
Non-Patent Literature 9: Henriksson, H. et al. Identification of cell proliferation zones, progenitor cells and a potential stem cell niche in the intervertebral disc region: a study in four species. Spine. 34, 2278-87 (2009).
Non-Patent Literature 10: Schmittwolf, C. et al. In vivo haematopoietic activity is induced in neurosphere cells by chromatin-modifying agents. EMBO J. 24, 554-66 (2005).
Non-Patent Literature 11: Shiota, M. et al. Isolation and characterization of bone marrow-derived mesenchymal progenitor cells with myogenic and neuronal properties. Exp Cell Res. 313, 1008-23 (2007).
Non-Patent Literature 12: Makinodan, E. et al. A novel role for Fyn: change in sphere formation ability in murine embryonic stem cells. Neuroscience. 147, 1-4 (2007).
Non-Patent Literature 13: Fujita, N. et al. CD24 is expressed specifically in the nucleus pulposus of intervertebral discs. Biochem Biophys Res Commun. 338, 1890-6 (2005).
Non-Patent Literature 14: Nagoshi, N. et al. Ontogeny and multipotency of neural crest-derived stem cells in mouse bone marrow, dorsal root ganglia, and whisker pad. Cell Stem Cell. 2, 392-403 (2008).
Non-Patent Literature 15: Lee, M.J. at al. In early development of the rat mRNA for the major myelin protein P(0) is expressed in nonsensory areas of the embryonic inner ear, notochord, enteric nervous system, and olfactory ensheathing cells. Dev Dyn. 222, 40-51 (2001).
Non-Patent Literature 16: Thompson, J.P. et al. Preliminary evaluation of a scheme for grading the gross morphology of the human intervertebral disc. Spine 15, 411-5 (1990).
Non-Patent Literature 17: Chan, C.K. et al. Endochondral ossification is required for haematopoietic stem-cell niche formation. Nature. 457, 490-4 (2009).
Non-Patent Literature 18: Arai, F. et al. Tie2/angiopoietin-1 signaling regulates hematopoietic stem cell quiescence in the bone marrow niche. Cell. 118, 149-61 (2004).
Non-Patent Literature 19: Sacco, A., Doyonnas, R., Kraft, P., Vitorovic, S. & Blau, H.M. Self-renewal and expansion of single transplanted muscle stem cells. Nature. 456, 502-6 (2008).
Non-Patent Literature 20: Masuda, H. et al. Noninvasive and real-time assessment of reconstructed functional human endometrium in NOD/SCID/gamma c (null) immunodeficient mice. Proc Natl Acad Sci U S A. 104, 1925-30 (2007).
Non-Patent Literature 21: Hara, T. et al. Suppression of basal autophagy in neural cells causes neurodegenerative disease in mice. Nature. 441, 885-9 (2006).
Non-Patent Literature 22: Lawson, D.A., Xin, L., Lukacs, R.U., Cheng, D. & Witte, O.N. Isolation and functional characterization of murine prostate stem cells. Proc Natl Acad Sci U S A. 104, 181-6 (2007).
Non-Patent Literature 23: Martinez, C., Hofmann, T.J., Marino, R., Dominici, M. & Horwitz, E.M. Human bone marrow mesenchymal stromal cells express the neural ganglioside GD2: a novel surface marker for the identification of MSCs. Blood. 109, 4245-8 (2007).
Non-Patent Literature 24: Xu, J. et al. Neural ganglioside GD2 identifies a subpopulation of mesenchymal stem cells in umbilical cord. Cell Physiol Biochem. 23, 415-24 (2009).
Non-Patent Literature 25: Androutsellis-Theotokis, A. et al. Targeting neural precursors in the adult brain rescues injured dopamine neurons. Proc Natl Acad Sci U S A. 106, 13570-5 (2009).
Non-Patent Literature 26Morikawa, S. et al. Development of mesenchymal stem cells partially originate from the neural crest. Biochem Biophys Res Commun. 379, 1114-9 (2009).
Non-Patent Literature 27: Aguiar, D.J., Johnson, S.L. & Oegema, T.R. Notochordal cells interact with nucleus pulposus cells: regulation of proteoglycan synthesis. Exp Cell Res. 246, 129-37 (1999).
Non-Patent Literature 28: Erwin, W.M., Ashman, K., O'Donnel, P. & Inman, R.D. Nucleus pulposus notochord cells secrete connective tissue growth factor and up-regulate proteoglycan expression by intervertebral disc chondrocytes. Arthritis Rheum. 54, 3859-67 (2006).
Non-Patent Literature 29: Watanabe, T. et al. Human nucleus pulposus cells significantly enhanced biological properties in a coculture system with direct cell-to-cell contact with autologous mesenchymal stem cells. J Orthop Res. 2009 Dec 1.
Non-Patent Literature 30Meisel, H.J. et al. Clinical experience in cell-based therapeutics: intervention and outcome. Eur Spine J 15:S397-405 (2006).
Non-Patent Literature 31: Hiyama, A. et al. Transplantation of mesenchymal stem cells in a canine disc degeneration model. J Orthop Res. 26, 589-600 (2008).
Non-Patent Literature 32: Sakai, D. et al. Differentiation of mesenchymal stem cells transplanted to a rabbit degenerative disc model: potential and limitations for stem cell therapy in disc regeneration. Spine. 30, 2379-87 (2005).
Non-Patent Literature 33: Hoogendoorn, R.J. et al. Adipose stem cells for intervertebral disc regeneration: current status and concepts for the future. J Cell Mol Med. 12, 2205-16 (2008).
Non-Patent Literature 34: Ganey, T. et al. Disc chondrocyte transplantation in a canine model: a treatment for degenerated or damaged intervertebral disc. Spine 28, 2609-20 (2003).
Non-Patent Literature 35: Gruber, H.E. et al. Autologous intervertebral disc cell implantation: a model using Psammomys obesus, the sand rat. Spine 27, 1626-33 (2002).
Non-Patent Literature 36: Pittenger, M. Sleuthing the source of regeneration by MSCs. Cell Stem Cell. 5, 8-10 (2009).
Non-Patent Literature 37: Sakai, D., Nakai, T., Mochida, J., Alini, M. & Grad, S. Differential phenotype of intervertebral disc cells: microarray and immunohistochemical analysis of canine nucleus pulposus and anulus fibrosus. Spine. 34, 1448-56 (2009).
Non-Patent Literature 38: Muguruma, Y. et al. In vivo and in vitro differentiation of myocytes from human bone marrow-derived multipotent progenitor cells. Exp Hematol. 31,1323-1330 (2003).
Non-Patent Literature 39: Nagoshi, N. et al. Ontogeny and multipotency of neural crest-derived stem cells in mouse bone marrow, dorsal root ganglia, and whisker pad. Cell Stem Cell. 2, 392-403 (2008).
Non-Patent Literature 40: Nakamura, Y. et al. Expression of CD90 on keratinocyte stem/progenitor cells. Br J Dermatol. 154, 1062-70 (2006).
Non-Patent Literature 41: Nakamura, Y. et al. Angiopoietin-1 supports induction of hematopoietic activity in human CD34- bone marrow cells. Exp. Hematol. 35, 1872-1883 (2007).
Non-Patent Literature 42: Kawada, H. et al. Rapid ex vivo expansion of human umbilical cord hematopoietic progenitors using a novel culture system. Exp. Hematol. 27, 904-915 (1999).
Non-Patent Literature 43: Nakamura, Y. et al. Ex vivo generation of CD34(+) cells from CD34(-) hematopoietic cells. Blood. 94, 4053-4059 (1999).
Non-Patent Literature 44: Wang, J. et al. SCID-repopulating cell activity of human cord blood-derived CD34- cells assured by intra-bone marrow injection. Blood. 101, 2924-2931 (2003).
Non-Patent Literature 45: Sakai, D., Nakai, T., Mochida, J., Alini, M. & Grad, S. Differential phenotype of intervertebral disc cells: microarray and immunohistochemical analysis of canine nucleus pulposus and anulus fibrosus. Spine. 34, 1448-56 (2009).

### SUMMARY OF INVENTION

### Problem to be Solved by the Invention

In recent years, the roles of endogenous stem cells and progenitor cells have attracted attention in the homeostasis of many organs. Moreover, there are many reports mentioning that micro-environment of the surrounding region, a so-called niche, is important in preserving the plasticity of the stem cells and their maintenance ^{8,9}. There have been no reports on detailed identification of the endogenous stem cells and localization of the niche regarding intervertebral disk tissues in the past. Moreover, in the same manner as hematopoietic stem cells that interact with osteoblasts, various intercellular adhesions and the involvement of related pathways are expected in the local surrounding region of mesenchymal stem cells as well.

The purpose of the present invention is to provide the isolated intervertebral disk NP stem cells and progenitor cells as well as the peripheral technology thereof, including a cultivation method, method of use, etc., through searching for a surface marker that identifies a cell population with stem cell characteristics in the intervertebral disk NP, and identifying the stem cell niche in the NP tissues and discovering the significance of the NP endogenous stem cells regarding age-related changes in the intervertebral disk in humans.

### Solution to Problem

The inventors of the present invention completed the present invention that solves the abovementioned problems based on the experimental outcomes indicated in the examples described later.

That is, the present invention provides an intervertebral disk nucleus pulposus cell (or the cell population comprising said cell) isolated from the intervertebral disk nucleus pulposus of vertebrates characterized by being positive for at least one surface marker from among Tie2 and GD2.

One aspect of said intervertebral disk nucleus pulposus cell is a stem cell that is at least Tie2-positive for the surface marker and possesses self-renewal ability as well as multipotency capable of differentiating into adipocytes, osteocytes, chondrocytes, or neurons (referred to as the "intervertebral disk nucleus pulposus stem cell" in the present invention). Among such stem cells, those which are GD2-negative for the surface marker are in a dormant state, and those which are GD2-positive are in an activated state.

Moreover, another aspect of said intervertebral disk nucleus pulposus cell is a progenitor cell that is Tie2-negative and GD2-positive for the surface marker, and capable of differentiating into adipocytes, osteocytes, chondrocytes, or neurons (referred to as the "intervertebral disk nucleus pulposus progenitor cell" in the present invention).

Regarding said intervertebral disk nucleus pulposus stem cell, the surface marker is additionally CD24-negative, CD44-positive/negative (in the case of stem cells) or positive (in the case of progenitor cells), CD271-positive, and Flt1-positive. Moreover, regarding said intervertebral disk nucleus pulposus progenitor cell, the surface marker is additionally CD24-negative or positive, CD44-positive, CD271-positive/negative or negative, and Flt1-positive/negative or negative.

Said vertebrates are preferably mammals, for example, a human or a mouse, as indicated in the example.

In one aspect, the present invention provides a cultivation method for the intervertebral disk nucleus pulposus cell (or the cell population including said cells), the method characterized by comprising: isolating a cell positive for at least one surface marker from among Tie2 and GD2 from a nucleus pulposus cell population collected from the intervertebral disk nucleus pulposus of the vertebrate or a cell population obtained by cultivating the same.

One aspect of said cultivation method of intervertebral disk nucleus pulposus cells is a cultivation method of the intervertebral disk nucleus pulposus stem cell wherein the isolated cell is at least Tie2-positive for the surface marker, and another aspect is a cultivation method of the intervertebral disk nucleus pulposus progenitor cell wherein the isolated cell is Tie2-negative and GD2-positive for the surface marker. The cultivation method of said intervertebral disk nucleus pulposus stem cell may comprise cultivating said cells in the presence of an angiopoietin I (Ang-1). The Tie2 is an receptor of angiopoietin I.

Furthermore, the present invention also provides a cultivation method of adipocytes, osteocytes, chondrocytes, or neurons comprising a step of culturing said intervertebral disk nucleus pulposus cell (stem cell and/or progenitor cell) under differentiation-inducing conditions.

In one aspect, the present invention provides a cell composition characterized by comprising said intervertebral disk nucleus pulposus cell (stem cell and/or progenitor cell). Regarding such a cell composition, for example, those for treatment or prevention of intervertebral disk disorders are preferable.

In one aspect, the present invention provides a treatment or prevention method of intervertebral disc disorders in vertebrates (excluding humans) comprising transplanting said intervertebral disk nucleus pulposus cells (stem cells and/or progenitor cells) or said cell composition on the intervertebral disk.

Moreover, the present invention provides a treatment or prevention method of intervertebral disk disorders in vertebrates (excluding humans) comprising administrating Ang-1 to the intervertebral disk nucleus pulposus stem cell in the intervertebral disks of the living body.

Furthermore, said treatment or prevention method of intervertebral disk disorders may be applied in the same manner to humans.

In one aspect, the present invention provides a method of obtaining an indicator related to the state of the intervertebral disk, comprising measuring the proportion of the intervertebral disk nucleus pulposus stem cells in a nucleus pulposus cell population sampled from the intervertebral disk nucleus pulposus of a vertebrate.

In the present specifications, the intervertebral disk nucleus pulposus stem cell and/or progenitor cell may be referred to as the "intervertebral disk nucleus pulposus stem cell/progenitor cell." Moreover, as commonly used in said technical field, a positive for the surface marker may be expressed by the symbol "+" while a negative may be expressed by the symbol "-" (for example, "Tie2+GD2+" means "Tie2-positive and GD2-positive").

### Advantageous Effects of Invention

According to the present invention, isolation, culturing, proliferation of the intervertebral disk nucleus pulposus stem cell/progenitor cell and treatments as well as prevention of intervertebral disk disorders become possible.

### BRIEF DESCRIPTION OF THE DRAWINGS

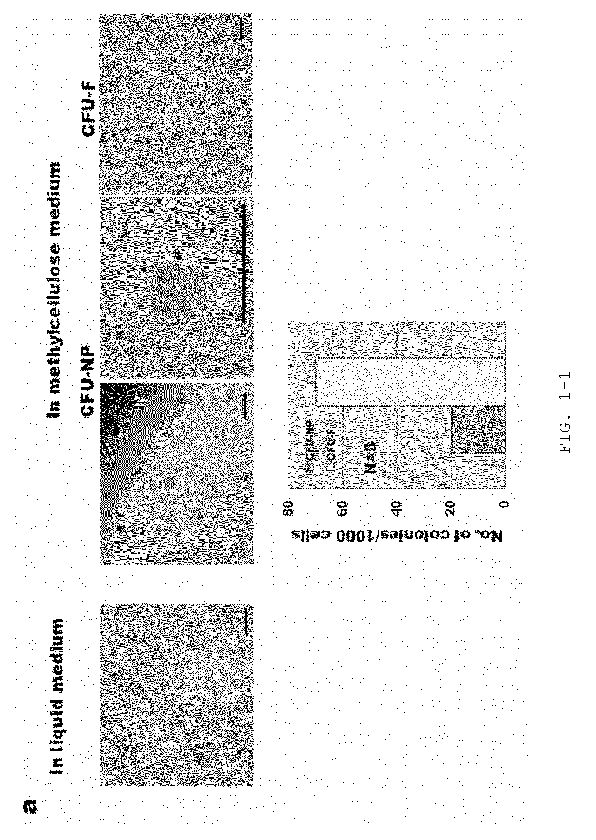

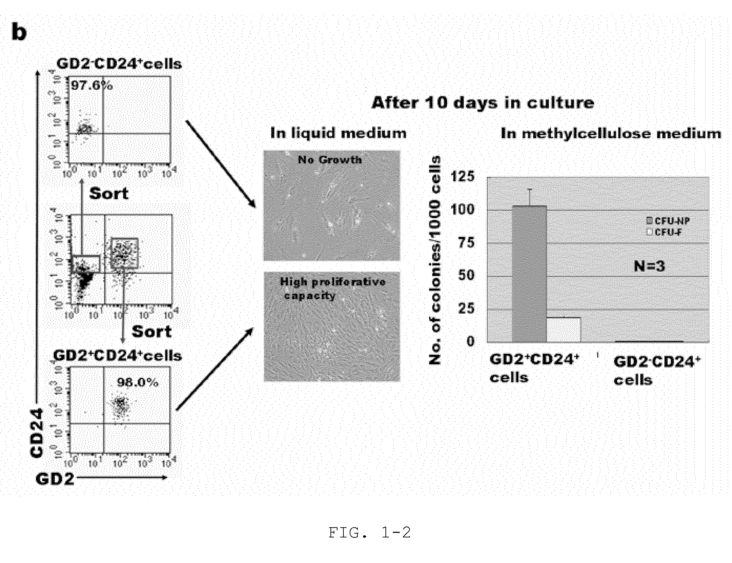

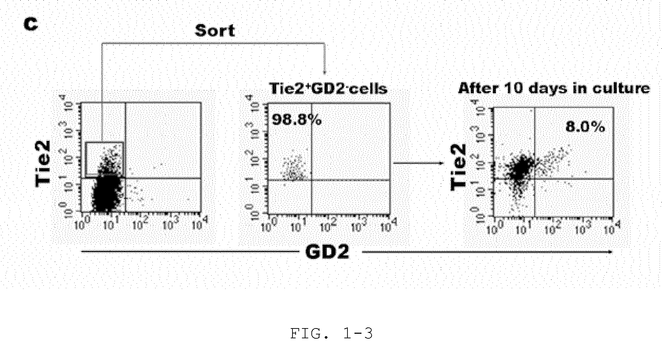

FIG. 1. Identification of mouse nucleus pulposus cell fractions by colony-forming ability. (a) Colony forming cells of nucleus pulposus (CFU-NP) and colony forming cells of fibroblast (CFU-F) in mouse primary nucleus pulposus cells. The scale bars are 100 µm. (b) The purification of CFU-NP cells using the surface markers GD2 and Tie2 from cultured nucleus pulposus cells. Said GD2+CD24+ or GD2-CD24+ cells purified by the surface marker were cultured for 10 days in a liquid culture medium or a methylcellulose medium. (c) The inducement of GD2+ cells from the purified Tie2+GD2- cells. The Tie2+GD2- cells were purified from the cultured nucleus pulposus cells, and additionally cultured for 10 days. The results are shown as a mean value ± standard deviation (SD).

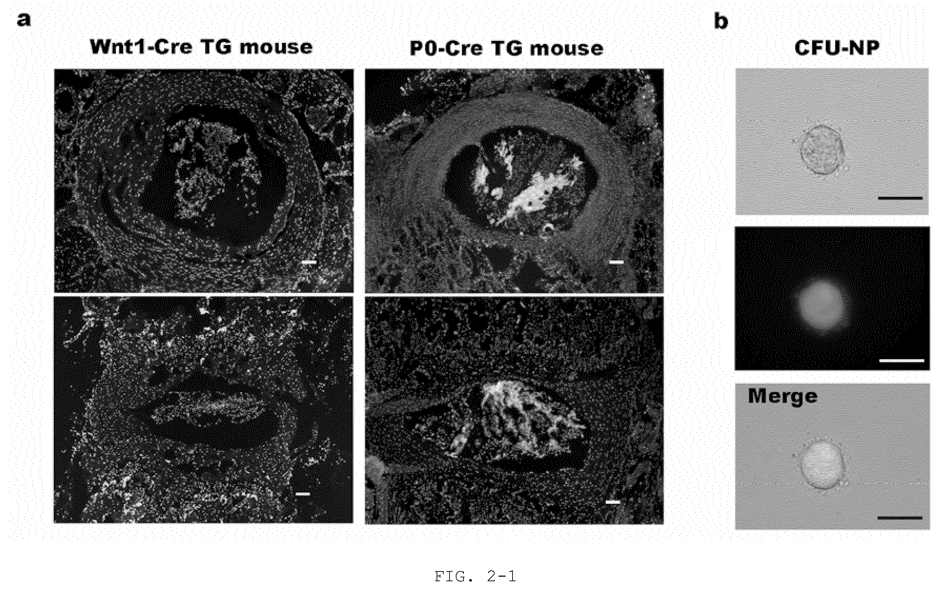

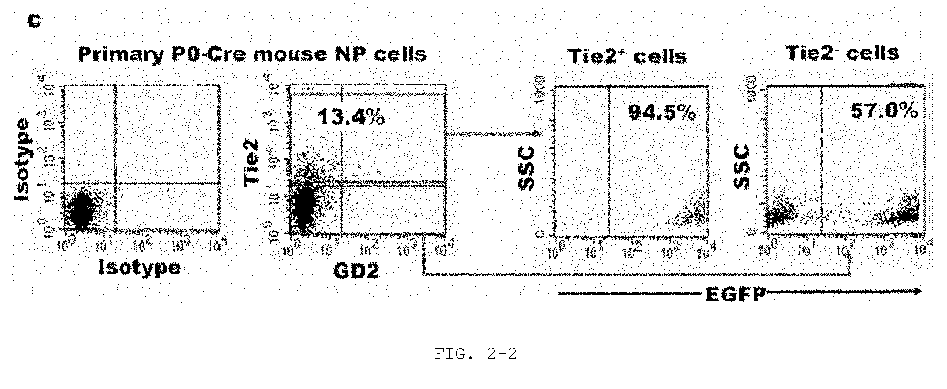

FIG. 2. Analysis of nucleus pulposus cells in P0-Cre and Wnt1-Cre/Floxed-genetically modified mice. (a) Although enhanced GFP-positive (EGFP)-positive nucleus pulposus cells were observed in the intervertebral disks of a P0-Cre/Floxed mouse as a result of immunostaining an anti-green fluorescent protein (GFP) of the intervertebral disks of the same mouse aged 16-weeks old, this was not observed in the intervertebral disks of a Wnt-Cre/Floxed mouse. The upper photograph panels show horizontal sections of an intervertebral disk of the mouse tailbone, while the lower photograph panels show sagittal plane sections. The nuclei is stained blue by 4'-6-Diamidino-2-phenylindole (DAPI). (b) The primary nucleus pulposus cells of the P0-Cre/Floxed mouse (6-weeks old) were cultured for 10 days in methylcellulose medium. The upper photograph shows a phase contrast microscope image of the shaped CFU-NP, while the middle shows a fluorescent microscope image. A CFU-NP colony emitting green fluorescence was observed (lower picture panel). The scale bars are 100 µm. (c) The results from analyzing the primary nucleus pulposus cells of the P0-Cre/Floxed mouse (6-weeks old) by a flow cytometry method.

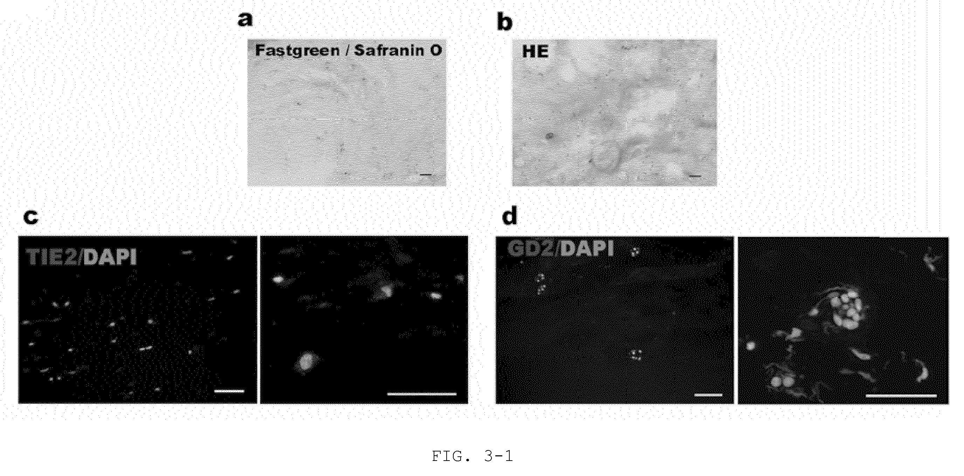

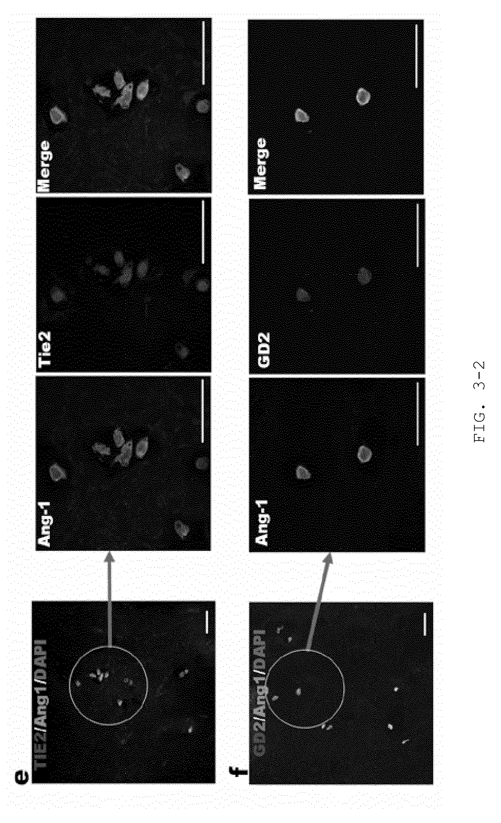

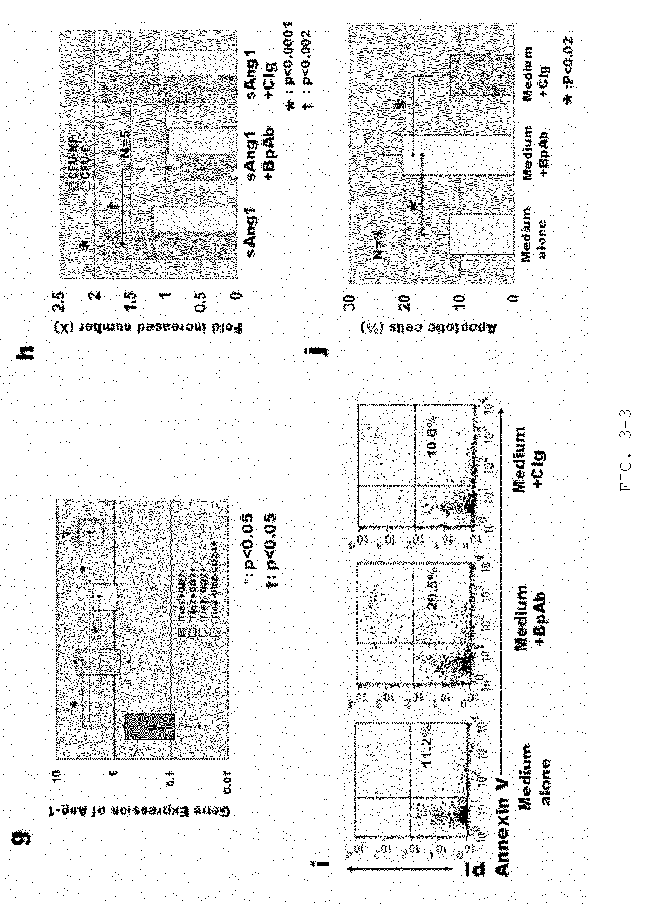

FIG. 3. Analysis of the human nucleus pulposus cell and the niche thereof. Human intervertebral disk-derived tissue was stained by (a) fast green and safranin O and (b) hematoxylin- eosin staining. The scattered nuclei or clustered nuclei shows the presence of the NP cells. (c) It may be understood from immunostaining that Tie2+ cells are present by scattering in the center of the nucleus pulposus tissue. (d) It may be understood that in contrast with the Tie2+ cells, GD2+ cells are present by shaping a cluster in the nucleus pulposus tissue. Due to double staining of Tie2 or GD2 and angiopoietin I (Ang-1), the presence of (e) Tie2+Ang-1+ cells or (f) GD2+Ang-1+ cells was confirmed. The nuclei is stained blue by DAPI staining. The scale bars are 50 µm. (g) Quantitative measurement of Ang-1 gene by a real-time PCR method in isolated nucleus pulposus cell fractionation. The expression level of each cell was expressed by an absolute number compared with the 18S level in the Tie2-GD2- cells (* p<0.05 indicates a significant difference in a comparison between each fractionated cell). † p<0.05 expresses those showing a significant difference compared to the Tie2-GD2- cells. (h) The supporting action of a water-soluble Ang-1 (sAng-1) against CFU-NP. The primary human nucleus pulposus cell was cultured in α-MEM culture medium without fetal bovine serum (FBS) for 7 days in the presence of 500 ng/ml human sAng-1 by adding 10µg/ml goat-derived human Tie2 neutralizing antibodies (BpAb) or a control goat immunoglobulin (CIg). After culturing was completed, a colony forming test was carried out on the nucleus pulposus cells. (i, j) Induction of apoptosis in the nucleus pulposus cells by anti-human Tie2 neutralizing antibodies (BpAb). The primary human nucleus pulposus cell was cultured in α-MEM culture medium without fetal bovine serum (FBS) for 2 days by adding 10µg/ml goat-derived human Tie2 neutralizing antibodies (BpAb) or the control goat immunoglobulin (CIg). Apoptotic cells were determined as annexin V positive and PI negative cell populations. The results are shown as a mean value ± SD.

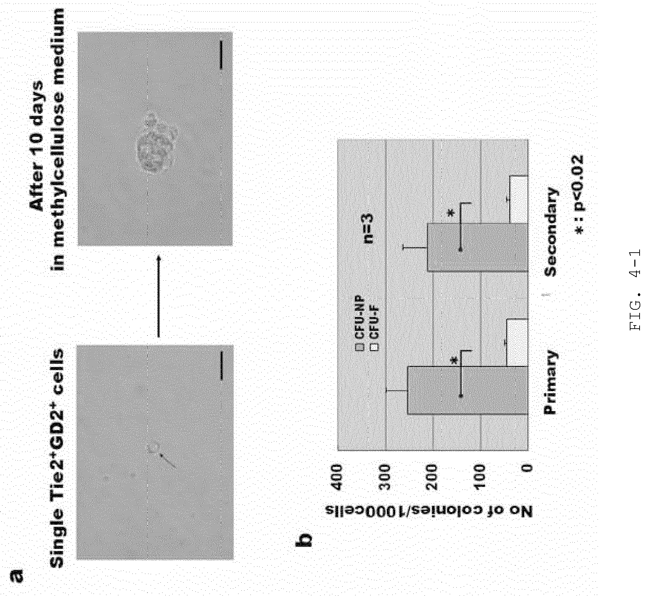

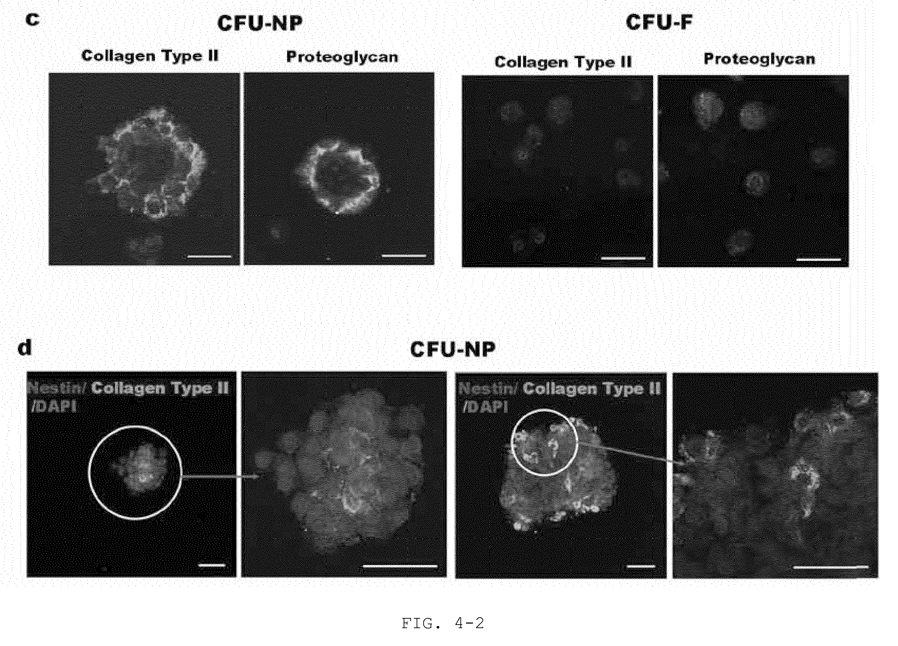

FIG. 4. Clonal analysis of the human nucleus pulposus cell.
   (a) Colony-forming ability derived from a single Tie2+GD2+ cell. The Tie2+GD2+ cells were purified from the cultured nucleus pulposus cells. Said cells were further purified and put in a 96-well culture plate one by one using a manual manipulator (Narishige) under a phase-contrast microscope. The individually placed Tie2+GD2+ cells were cultured in 100µL methylcellulose medium for 10 days and subsequently shaped a single CFU-NP colony.
   (b) The primary and secondary colony-forming ability of the purified Tie2+GD2+ cells. A single cell derived from the CFU-NP colony shaped from the primary Tie2+GD2+ cells were cultured in methylcellulose medium for 10 days, and subsequently CFU-NP colonies and CFU-F colonies were shaped again. (c) The results from immunostaining the CFU-NP colonies and CFU-F colonies shaped by the Tie2+GD2+ cells. The generation of collagen type II and proteoglycan was observed in the CFU-NP colonies by immunostaining of the cytospin specimen of these colonies. In the CFU-F colonies, the generation of collagen type II and proteoglycan was negative or very low. (d) It was confirmed that the CFU-NP colonies expressing collagen type II was expressing nestin by double immunostaining. The expression of nestin was observed as coexpression with collagen type II generating cells or a mosaic expression of both. The nuclei is stained blue by DAPI immunostaining. The scale bars are 50 µm. The results are shown as a mean value ± SD.

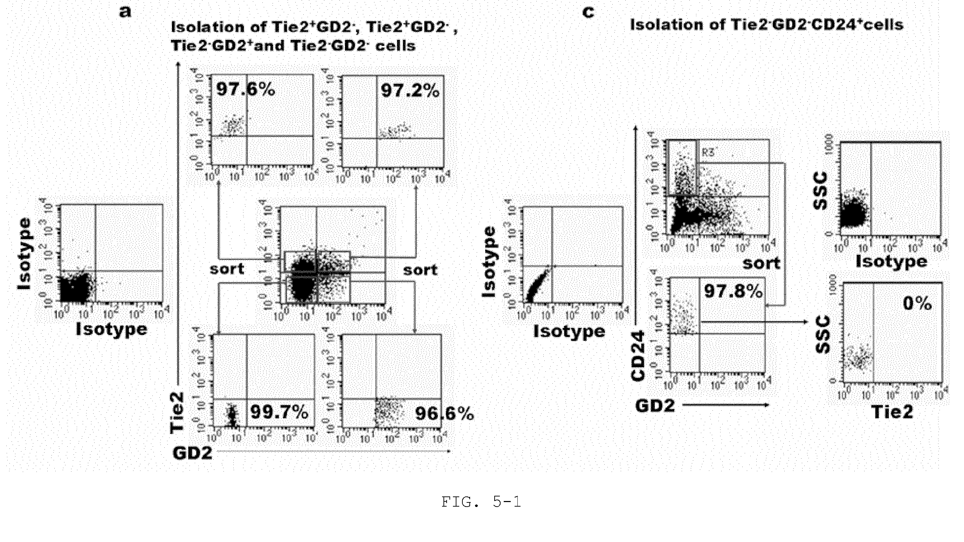

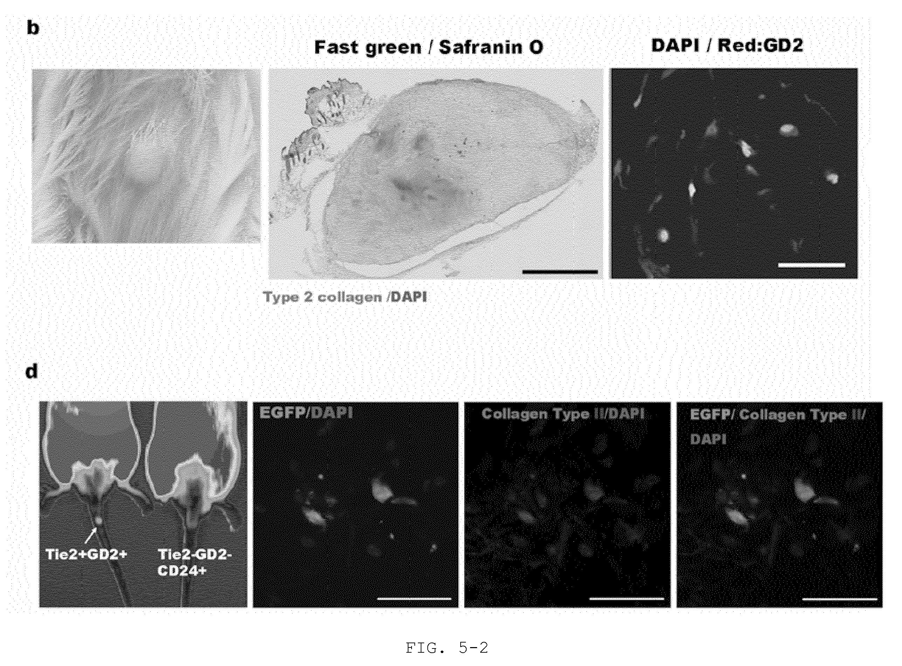

FIG. 5. Transplantation of a human nucleus pulposus cell into an immunodeficient mouse. (a) Purification of Tie2+GD2-, Tie2+GD2+, Tie2-GD2+ and Tie2-GD2- cells was carried out from the nucleus pulposus cells derived from a young donor (18 to 25 years old) cultured for 7 to 10 days. (b) The engraftment of the Tie2+GD2+ cells 8 weeks after subcutaneous transplantation into the NOD/SCID mouse (left). The cyst generated after the engraftment was extirpated, followed by making a frozen section and staining it with fast green and Safranin O (middle). Identification of the GD2-positive cells from innunostaining and DAPI in the same section (right). The nuclei was stained blue by DAPI immunostaining. The black scale bars are 500 µm. The white scale bars are 50 µm. (c) Regarding purification of the Tie2-GD2-CD24+ cells, a nucleus pulposus from the same donor cultured in liquid for 20 to 25 days was used. purification of the Tie2+GD2+ cells was carried out as shown in FIG. 5 (a). (d) The enhanced green fluorescent protein-labeled Tie2+GD2+ and the Tie2-GD2-CD24+ human nucleus pulposus cells were transplanted into injured caudal intervertebral disks of NOD/SCID mice. The caudal vertebra of the same mice was measured by a bioluminescent technique 12 weeks after transplantation. As a result, the presence of the transplanted cells was confirmed in the group with the Tie2+GD2+ cell transplanted while it was confirmed to have disappeared in the Tie2-GD2-CD24+ cell transplanted group. In the nucleus pulposus region of the caudal intervertebral disk of the mouse confirmed with engraftment of said transplanted cell, the presence of EGFP-positive and collagen type II-positive cells was observed as a result of immunostaining. A typical section observed with engraftment is shown. The scale bars are 50 µm.

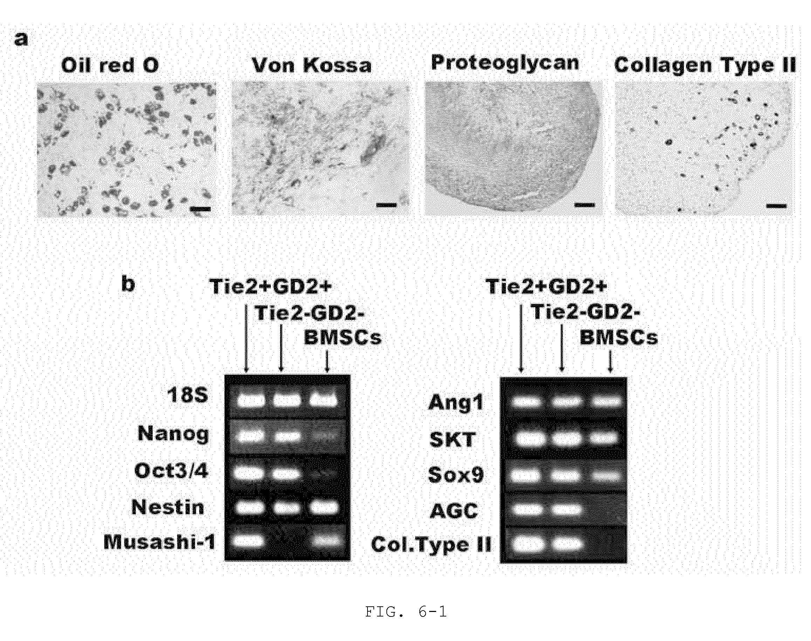

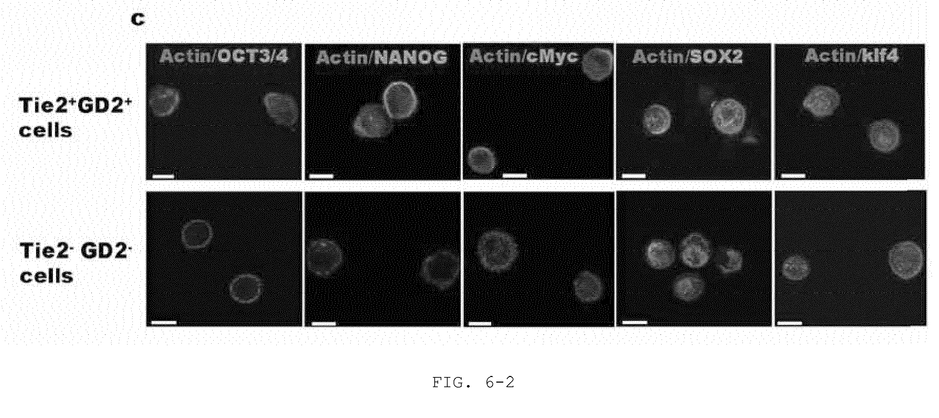

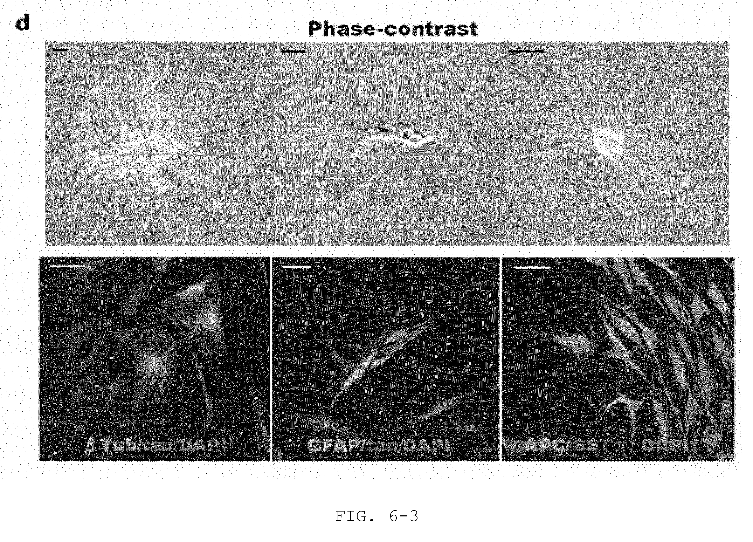

FIG. 6. Multipotency of the human nucleus pulposus Tie2+GD2+ cell. (a) The differentiation of the human nucleus pulposus Tie2+GD2+ cells into adipocytes, osteocytes, and chondrocytes. Nucleus pulposus cells were cultured for differentiation for 20 days by a culture inducing differentiation into adipocytes, osteocytes, and chondrocytes. After culturing was completed, the presence of proteoglycan and collagen type II was confirmed by oil red O and von Kossa staining as well as immunostaining using 3,3'-diaminobenzidine (DAB). The scale bars are 100 µm. (b) An analysis of purified human nucleus pulposus cells and bone marrow stroma cells using a semi-quantitative RT-PCR method. The mRNAs expressed in embryonic stem cells (ES) such as Nanog, Oct3/4, Nestin, Musashi-1 were detected in the NP cells. Furthermore, the expression of mRNAs of NP cell-specific SKT, Sox9, AGC, and collagen type II was detected in the NP cells. (c) The expression of Oct4, Nanog, cMyc, klf4 and Sox2 proteins in the Tie2+GD2+ and Tie2-GD2-nucleus pulposus cells with immunostaining. Each cells were adhered to a slide glass at a density of 8x10² /cm² and then fixed with 4% paraform-aldehyde. Subsequently, the ES markers of each cells were detected by Alexa Fluor-350-conjugated secondary antibodies in order to prevent any effect from the two types of fluorescence used for purification. The subject protein is shown in green while a cytoplasm actin is shown in red. The scale bars are 20 µm. (d) The human Tie2+GD2+ cultured nucleus pulposus cell is positive for nerve and glial cell markers. The presence of cells positive for β-Tubulin, tau, GFAP, GSTpi and adenomatous polyposis coli tumor suppresser gene (APC), including an phenotypic characteristic of neurons cells or glial cells, was confirmed. The results of double labeled immunofluorescent staining are shown. The nuclei is stained blue by DAPI immunostaining. The black and white scale bars are 50 µm.

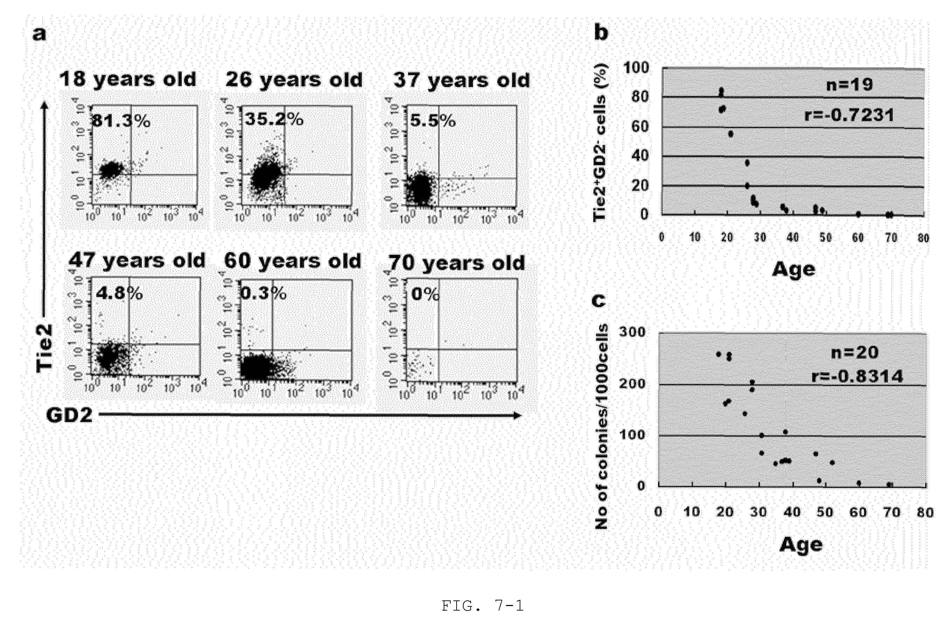

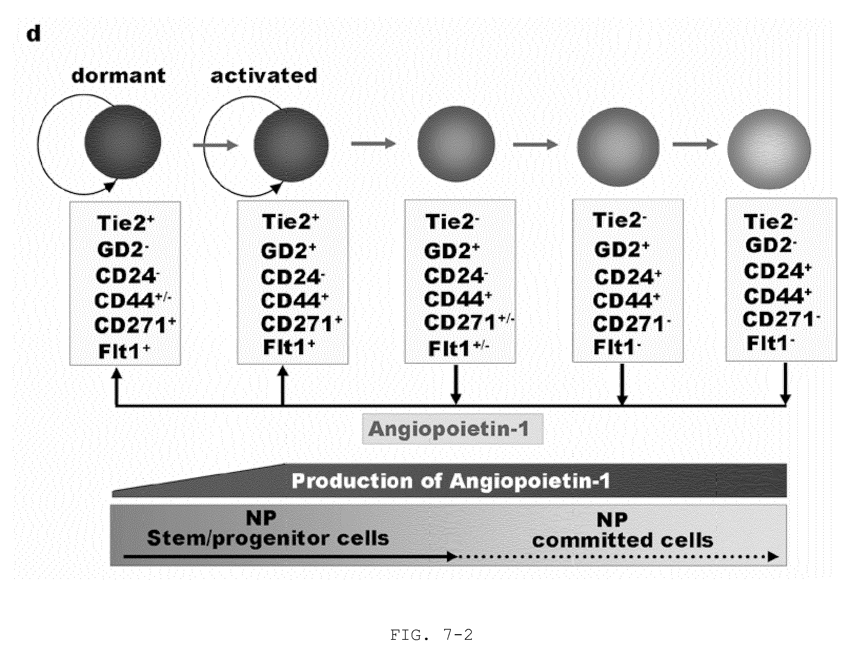

FIG. 7. Nucleus pulposus Tie2+ cells and CFU-NP decline with age. (a) Expression of the Tie2 and DG2 in nucleus pulposus cells of patients of various ages. (b) The relation between the proportion of Tie2+GD2- cells in the primary nucleus pulposus tissues and age. (c) The relation between the CFU-NP count in the primary nucleus pulposus tissues and age. (d) The hierarchy differentiating model of nucleus pulposus cells based on past results.

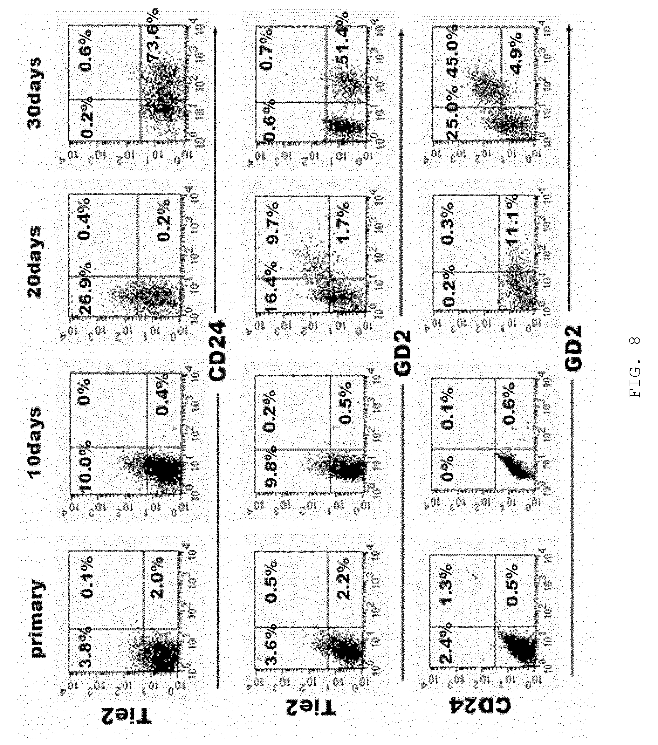

FIG. 8. Changes in Tie2, GD2, and CD24 differentiation in the mouse nucleus pulposus cell following liquid culturing. Expression of the Tie2+GD2+ or GD2+CD24+ cells was observed in the liquid culture of the mouse nucleus pulposus cells; however, expression of the Tie2+CD24+ cells was not observed. Tie2-GD2-CD24+ cells were observed after 30 days of culturing. Three representative examples from among the experimental results are shown.

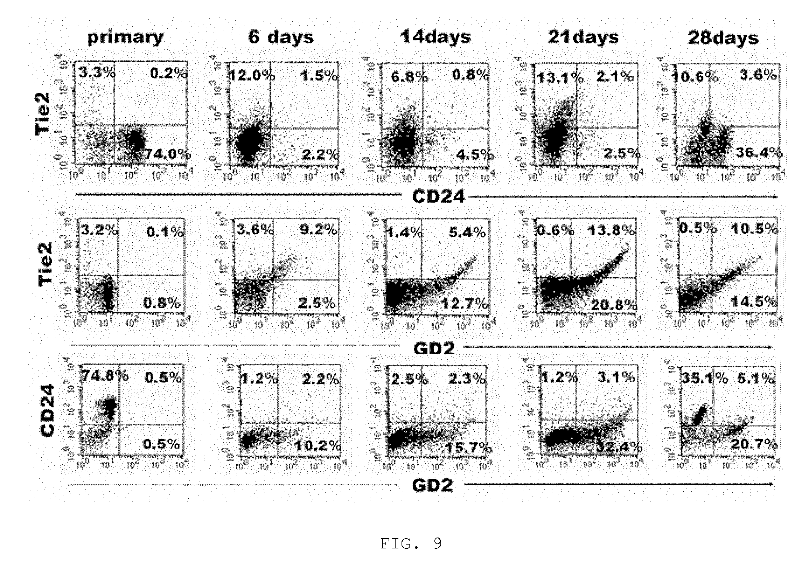

FIG. 9. Expressions of the Tie2, GD2, and CD24 molecules in the human nucleus pulposus cell following liquid culturing. In the human nucleus pulposus cells, an increase in the Tie2+GD2+ cells and Tie2-GD2+ cells was observed following liquid culturing, though the Tie2+GD2- cells did not increase. Meanwhile, although the Tie2-GD2-CD24+ cells accounted for 70% or more of the primary nucleus pulposus cell, these disappeared after 6 days of culturing. However, 35% of the cells became Tie2-GD2-CD24+ cells again after 28 days of culturing.

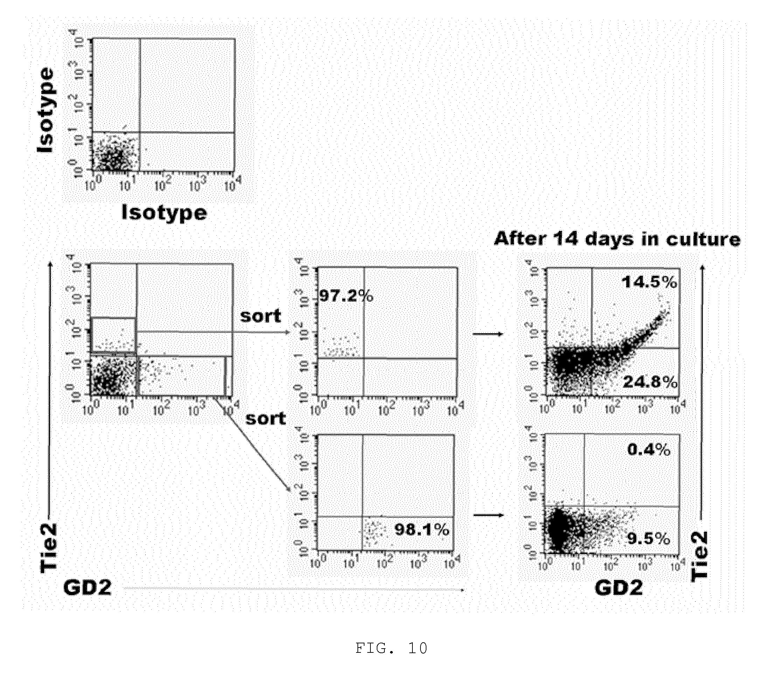

FIG. 10. GD2+ cells were induced from Tie2+ cells, but Tie2+ cells were not induced from GD2+ cells. The Tie2+ GD2- cells and the Tie2-GD2+ cells were purified from the human primary nucleus pulposus cells. Regarding the cell fractionation of each, liquid culturing was carried out and a change in the expressions of Tie2 and GD2 molecules was observed. Following 14 days of culturing, induction of the Tie2+GD2+ cells and Tie2-GD2+ cells was observed from the Tie2+GD2- cells; however, the induction of the Tie2+GD2+ cells was not confirmed from the Tie2-GD2+ cells. Five representative examples from among the experimental results are shown.

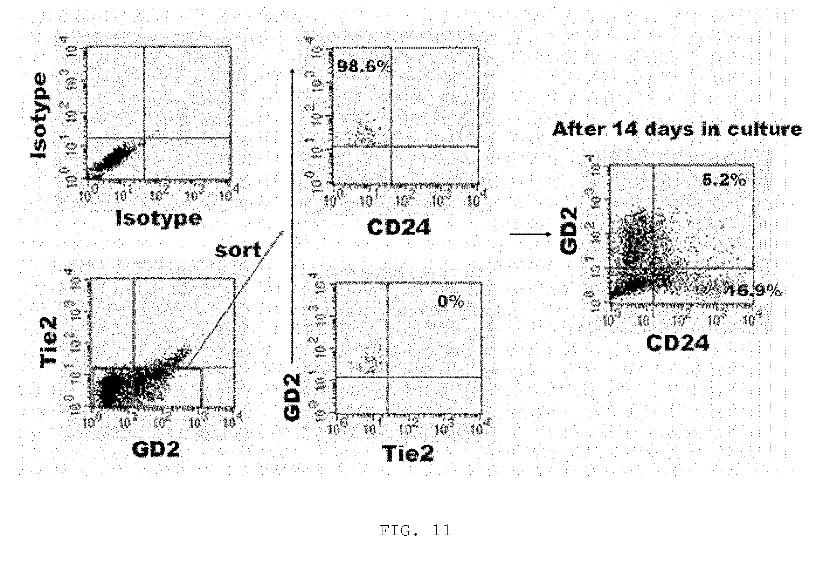

FIG. 11. CD24+ cells were induced from GD2+ cells. The Tie2-GD2+CD24- cells were purified from the human nucleus pulposus cell after 7 days of liquid culturing. When culturing was continuously carried out for 14 days, the Tie2-GD2+CD24- cells induced Tie2-GD2+CD24+ and Tie2-GD2-CD24+ cell fractionation. Five representative examples from among the experimental results are shown.

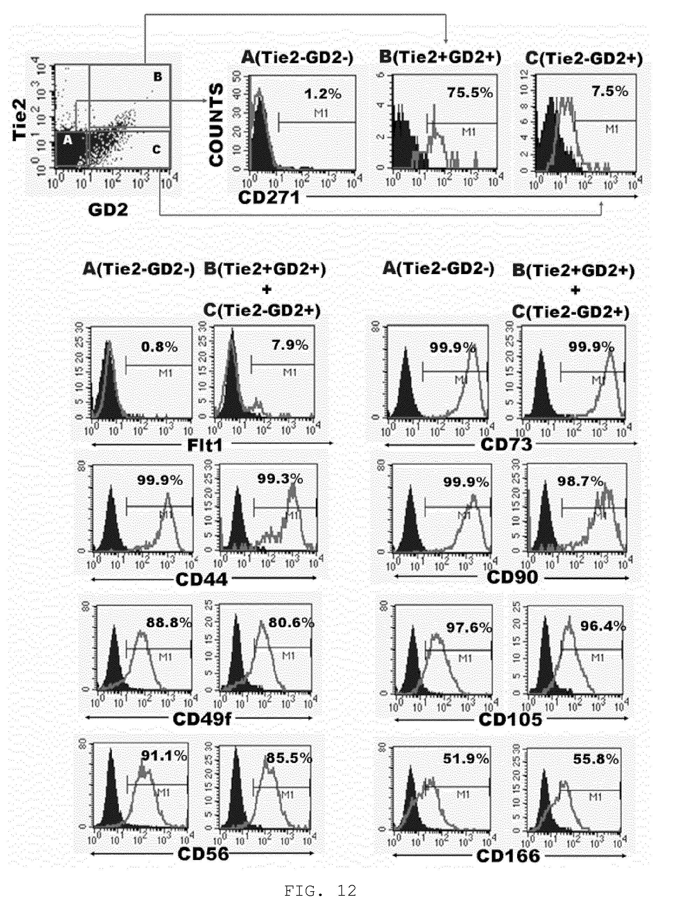

FIG. 12. Expression of other surface molecules in the human nucleus pulposus cell. The expression of a bone marrow stroma cell-related surface molecule was investigated using the human nucleus pulposus cell. The expressions of the Tie2, GD2, and other surface molecules were investigated regarding the nucleus pulposus cells that underwent and completed 7 days of liquid culturing. Moreover, said other surface molecules were investigated by three fractionations from among A (Tie2-GD2-), B (Tie2+GD2+), and C (Tie2-GD2+). As a result, CD271 was significantly expressed in the B fractionation, while the expression thereof was observed in part of the C fractionation, and the expression thereof was not observed in the A fractionation. Flt1, which is a VEGF-A receptor, was expressed in part of B and C fractionations, but was not observed in the A fractionation. Moreover, expressions of CD44, CD49f, CD56, CD73, CD90, and CD105 were observed in 80% or more A, B, and C fractionations, and furthermore, in 50% or more of each, expression of CD166 was observed. Five representative examples from among the experimental results are shown.

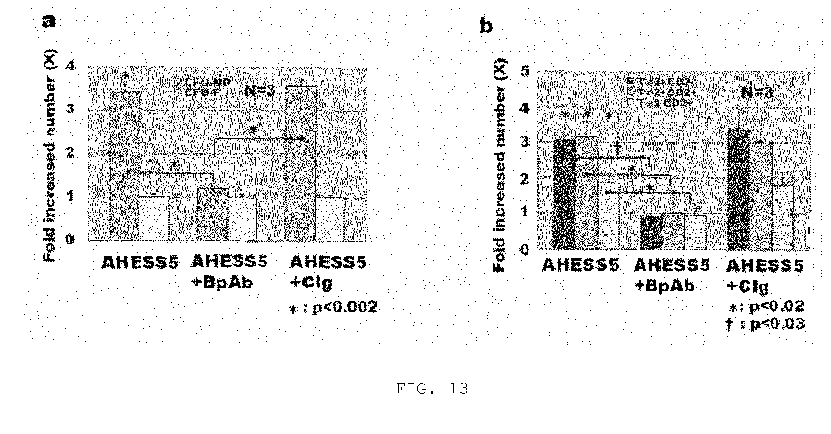

FIG. 13. Mixed culture of mouse stromal cells generating human Ang-1, AHESS-5 cells and human nucleus pulposus cells. (a) As a result of mixed culturing of 7 days, the AHESS-5 cells caused a significant increase in the CFU-NP count. However, no increase in the CFU-F count was caused. The effect of said AHESS-5 cells was hindered by adding an anti-Tie2 neutralizing antibodies. (b) Moreover, the AHESS-5 cells caused an increase in the Ti2+GD2-cells and Tie2+GD2+ cells. Said effects were hindered by adding the anti-Tie2- neutralizing antibodies in the same manner as the previous result.

### DESCRIPTION OF EMBODIMENTS

### • Intervertebral disk nucleus pulposus stem cell/progenitor cell

The intervertebral disk nucleus pulposus cell, which is the subject of the present invention, is isolated from the intervertebral disk nucleus pulposus of the vertebrate and is positive for at least one surface marker among Tie2 and GD2, wherein the stem cell and the progenitor cell are included. The mature intervertebral disk nucleus pulposus cell that finished differentiating is negative for both Tie2 and GD2.

The intervertebral disk nucleus pulposus stem cell is at least Tie2-positive for the surface marker, and is a stem cell possessing self-renewal ability and multipotency allowing differentiation into at least adipocytes, osteocytes, chondrocytes, or neurons (possible differentiation into other cells is not ruled out). Said stem cell is GD2 negative in the dormant state, but said stem cell is GD2 positive when activated. Various stimulations may be considered for activating the stem cell in the dormant state. For example, isolating the cell in the dormant state from the tissue and subculturing this may be raised as one technique. Meanwhile, the intervertebral disk nucleus pulposus progenitor cell is Tie2 negative and GD2 positive for the surface marker, and is a progenitor cell capable of differentiation into any among adipocytes, osteocytes, chondrocytes, or neurons. The intervertebral disk nucleus pulposus cell including said intervertebral disk nucleus pulposus stem cell/progenitor cell is a notochord-derived cell.

The "vertebrate", from which the intervertebral disk nucleus pulposus cell of the present invention is derived, is not particularly limited as long as it comprises the intervertebral disk nucleus pulposus, but is preferably a mammal, including humans and mammals other than humans (for example, mice, rats, pigs).

The intervertebral disk nucleus pulposus stem is characterized by, for example, CD24 negative, CD44 positive/negative (in case of the stem cell) or positive (in case of the progenitor cell), CD271 positive and Flt1 positive for the surface marker, as well as said Tie2 and GD2. Meanwhile, the intervertebral disk nucleus pulposus progenitor cell is characterized by, for example, CD24 negative or positive, CD44 positive, CD271 positive/negative or negative and Flt1 positive/negative or negative for the surface marker, as well as said Tie2 and GD2 (refer to FIG. 7d).

### • Cultivation method

The intervertebral disk nucleus pulposus stem cell/progenitor cell or the cell population comprising said cells of the present invention may be cultured and made representatively by using a cultivation method that comprises the isolation of cells positive for at least one surface marker from among Tie2 and GD2 from the nucleus pulposus cell population collected from the intervertebral disk nucleus pulposus of the vertebrate or the cell population obtained by culturing this; that is, the intervertebral disk nucleus pulposus stem cells with the surface marker being at least Tie2-positive and/or the intervertebral disk nucleus pulposus progenitor cells being Tie2-negative and GD2-positive.

The intervertebral disk nucleus pulposus cells may be separated from nucleus pulposus of intervertebral disk, which is collected from the vertebrate, followed by carrying out treatment such as digesting, dispersing, washing, etc. The cultivation method of the intervertebral disk nucleus pulposus cells is publicly known, and culturing should be done under an appropriate culture medium and conditions.

Whether the Tie2, GD2, and other surface markers are positive or negative may be determined from publicly-known methods such as cell-staining using a fluorescent antibody (preferably a monoclonal antibody, but a secondary antibody may be concomitantly used as needed) corresponding to the protein as the marker. The intervertebral disk nucleus pulposus stem cells/progenitor cells may be purified by fractionating a cell population of the given marker type from among the cell population stained in aforesaid manner with the use of an apparatuse such as a flow cytometer and a cell sorter (a cell isolating apparatus).

By means of further culturing and proliferating the intervertebral disk nucleus pulposus stem cells/progenitor cells purified in this manner, a cell population with high purity comprising many intervertebral disk nucleus pulposus stem cells/progenitor cells may be made.

In the present invention, the "isolated" intervertebral disk nucleus pulposus stem cells/progenitor cell (population) refers to the intervertebral disk nucleus pulposus stem cells/progenitor cell (population) taken from the vertebrate body, becoming available for various usages. It particularly refers to those that can be distinguishable from other nucleus pulposus cells (population) by the surface marker (stem cell: at least Tie2 positive; progenitor cell: Tie2 negative and GD2 positive). The cell population comprising the intervertebral disk nucleus pulposus stem cell/progenitor cell of the present invention should have a purity suitable for each of the various usages (culturing for proliferation, preparing a cell composition, etc.), and it is not necessarily required that said cell population not contain any other cells (Tie2 negative and GD2 negative cells which are differentiated from the intervertebral disk nucleus pulposus stem cell/progenitor cell and matured, etc.). For example, the purity regarding any of the intervertebral disk nucleus pulposus stem cells, the intervertebral disk nucleus pulposus progenitor cells, or the total thereof should be higher than that of a normal intervertebral disk nucleus pulposus cell population in vivo, preferably 90% or more and further preferably 95% or more purity. Said maximum purity can be brought close to 100%, although it may be 99%, 98%, or 97%.

Another aspect of the cultivation method of the intervertebral disk nucleus pulposus stem cell of the present invention comprises a step of culturing said cells in the presence of angiopoietin I. Such a cultivation method may be applied when culturing the nucleus pulposus cell population collected from the intervertebral disk nucleus pulposus of the vertebrate in order to isolate the intervertebral disk nucleus pulposus stem cells, or may be used to culture the already isolated and purified intervertebral disk nucleus pulposus stem cells.

The method of having angiopoietin I present in the cell culture environment is not particularly limited; angiopoietin I (preferably soluble in water) may be added to the culture solution, or cells generating angiopoietin I may be cultured together.

When the intervertebral disk nucleus pulposus stem cells are cultured in coexistence with angiopoietin I in sufficient concentration, said cells may be maintained and proliferated. On the contrary, if angiopoietin I is not present in the culture system, or if a substance inhibiting angiopoietin I activity is present even when angiopoietin I is present, the intervertebral disk nucleus pulposus stem cells cannot be maintained and the cells thereof eventually approach apoptosis.

The cultivation method of the adipocytes, osteocytes, chondrocytes, or neurons (neuron, glia) of the present invention comprises a step of culturing the intervertebral disk nucleus pulposus stem cell/progenitor cell under differentiation-inducing conditions.

Differentiation-inducing conditions of the adipocytes, osteocytes, chondrocytes, and neurons are publicly known (for example, see the example described later and references 42, 43, 44 from non-patent literature), and when wishing to differentiate from the intervertebral disk nucleus pulposus stem cell/progenitor cell to these cells, culturing by the respective appropriate differentiation-inducing conditions may be carried out such as exposing to a predetermined differentiation-inducing substance. The adipocytes, osteocyte, chondrocytes, and neurons made in this manner may be used for transplantation.

### • Cell composition

The cell composition of the present invention comprises said intervertebral disk nucleus pulposus stem cells and/or progenitor cells. Said cell composition is particularly suitable for the treatment or prevention of intervertebral disk disorders (lower back pain, intervertebral disk degeneration diseases, etc.).

That is, by means of transplanting said cells or cell composition to the nucleus pulposus of the injured or degenerated intervertebral disk and the transplanted intervertebral disk nucleus pulposus stem cells/progenitor cells and the cells differentiated from these generating an ECM (type II collagen, proteoglycan), restoration of the intervertebral disk tissues and maintenance of the reconstructed tissues becomes possible.

Depending on the use, said cell composition may, for example, further comprise components other than the intervertebral disk nucleus pulposus stem cells/progenitor cells such as a treatment drug, components suitable for culturing, proliferation, and differentiation of the cell, or already-differentiated adipocytes, osteocyte, chondrocytes, and neurons, or artificial substrates that become a scaffold for the cell. Moreover, the quantity and purity of the intervertebral disk nucleus pulposus stem cell/progenitor cell comprised in the cell composition may be appropriately adjusted according to use. Said cell composition, for example, is preferably prepared as a pharmaceutical composition formulated such that it may be injected into the intervertebral disk.

### • Treatment method, etc.

The treatment or prevention method of the intervertebral disk disorder by the present invention comprises, in one aspect, transplanting said intervertebral disk nucleus pulposus stem cells and/or progenitor cells or cell composition into the intervertebral disk. The quantity and purity of the intervertebral disk nucleus pulposus stem cells/progenitor cells used for transplantation or component of the cell composition and the proportion thereof may be appropriately adjusted according to the mode of the treatment and prevention.

In another aspect, the treatment or prevention method of the intervertebral disk disorder by the present invention comprises administrating angiopoietin I to the intervertebral disk nucleus pulposus stem cells (it may be transplanted as mentioned above or naturally present in vivo) in the intervertebral disk nucleus pulposus. This method corresponds to so-called cytokine therapy. As mentioned above, angiopoietin I is a protein (cytokine) necessary for the maintenance and proliferation of the intervertebral disk nucleus pulposus stem cells. Most cells configuring the intervertebral disk tissues express angiopoietin I, and when expression level thereof declines due to the decline of said cells, the intervertebral disk nucleus pulposus stem cell also declines, potentially eventually causing intervertebral disk disorders. Accordingly, by administrating a sufficient amount of angiopoietin I to the intervertebral disk nucleus pulposus stem cells, a decline in intervertebral disk nucleus pulposus stem cells may be prevented or the declining speed may be reduced, thereby allowing the intervertebral disk disorder to be treated or prevented. The angiopoietin I dosage may be appropriately adjusted according to the mode of treatment or prevention.

The method of obtaining an indicator related to the state of the intervertebral disk by the present invention comprises measuring the proportion of the nucleus pulposus stem cells in the nucleus pulposus cell population sampled from the intervertebral disk nucleus pulposus of the vertebrate.

The proportion of the intervertebral disk nucleus pulposus stem cells changes with the degree of intervertebral disk degeneration or the degree of aging, with the proportion tending to generally decline as the degree thereof advances. Accordingly, by measuring the proportion of the intervertebral disk nucleus pulposus progenitor cell population (Tie2+) in the nucleus pulposus cell population sampled from the intervertebral disk nucleus pulposus of the test subject via surface marker analysis using, for example, flow cytometry or a PCR method and carrying out statistical treatment using reference data, the indicator related to the state of intervertebral disk of said test subject may be obtained. The appropriate reference data and statistical approach should be prepared or selected according to the mode of analysis. For example, a standard value related to the degree of degeneration of the intervertebral disk or the mean value and standard deviation per age group is determined in advance, and by comparing the proportion of the intervertebral disk nucleus pulposus stem cell measurement regarding a certain sample with the standard value or mean value and standard deviation thereof, it is believed that the degree of degeneration of the intervertebral disk or whether it is in a normal range as a state of the intervertebral disk in the age range thereof, may be understood.

### EXAMPLES

### Materials and Method

### [1] Mice

P0-Cre/Floxed-EGFP mice and Wnt-Cre/Floxed-EGFP mice were obtained according to the method mentioned in the previous paper ¹⁴. Nonobese diabetic/severe combined immunodeficient mice (NOD/SCID) used as recipients of the transplant experiment were purchased from Clair Japan. These mice were raised in an isolator in the animal experiments laboratory at the Tokai University School of Medicine, and were provided for the experiment. Animal experiments using these mice were carried out with the approval of the animal experiment committee of Tokai University.

### [2] Collecting the mouse nucleus pulposus cells

The mouse NP tissue was collected from the caudal intervertebral disk under a surgical microscope. Simply, after removing the caudal epidermis and soft tissues, an incision was made between the cephalic endplate and the intervertebral disk and the intervertebral disk was collected. Subsequently, the gelatin-like NP tissues alone were collected using a delicate spoon appliance.

### [3] Human nucleus pulposus tissues

Upon carrying out this experiment, approval was obtained from the Institutional Ethics Review Board of Tokai University Hospital regarding the use of human samples. Moreover, upon providing the nucleus pulposus tissues, the patients were selected for the experiment upon sufficient explanation and obtaining consent from the patients. The breakdown of symptoms was: 10 cases of bursting fracture of the vertebra, 8 cases of hernia of lumbar intervertebral disk, and 2 cases of spondylolysis, totaling 20 cases. The age of patients was 18 to 70 years old, and the denaturing degree of the intervertebral disk in respective cases was measured based on the criteria set by Thompson et al¹⁶. The nucleus pulposus tissues were separated while carefully avoiding a fiber ring following extirpation of the lumbar intervertebral disk by surgery. Regarding the bursting fracture of the vertebra, if the upper cephalic endplate of the vertebra was fractured and the lower cephalic endplate was healthy, it was assumed that the site from the healthy cephalic endplate to the intervertebral disk was fundamentally a healthy site, and the NP tissues were collected. However, regarding the case of a strongly degenerated intervertebral disk, distinguishing between an NP tissues and an internal fiber ring was difficult; accordingly, the internal region of the nucleus pulposus tissues alone was collected as the nucleus pulposus tissues. The bone marrow aspirate of patients was collected during intervertebral disk surgery after sufficient explanation was given and consent was obtained from the patients. Induction of mesenchymal cells derived from the bone marrow of patients used for RT-PCR analysis was carried out by a standard method from the collected bone marrow aspirate³⁶.

### [4] Cell separation and culturing

The collected human or mouse nucleus pulposus tissues were cut up into small pieces using scissors, etc. The human nucleus pulposus tissues were digested for 1 hour by TrypLE Express (GIBCO), which is a protein-digesting enzyme, and for 2 hours by 0.25 mg/ml collagenase P (Roche). Subsequently, this was washed 2 times in an α-MEM culture medium (GIBCO) and inoculated at a cell density of 3, 000 to 5, 000 cells/cm². The same process as the human nucleus pulposus tissues was carried out on the mouse nucleus pulposus cells except for the fact that the digestion time by said digestive enzymes was 30 minutes each. The human and mouse nucleus pulposus cells isolated by digesting, dispersing, and washing were respectively cultured under 37°C, 5% CO₂ and 5% O₂ conditions in the same medium with 10% FBS (Cell culture Bioscience) added. Regarding the experimental method of in vitro multipotency of the nucleus pulposus cell into osteocytes, chondrocytes and adipocytes, refer to the column of "multipotency of the nucleus pulposus cell" described later [13].

### [5] Mixed culture

The nucleus pulposus cells that completed initial culturing in a single layered state of 4 days in a 10% FBS-added αMEM culture medium were used for the mixed culture. A cell culture insert for a 6-hole culture dish (BD Bioscience) with a polyethylene terephthalate film with a 0.4 µm pore size affixed to the bottom and a 6-hole culture dish were used for culturing. Specifically, 1.0x10⁴ NP cells were put in a cell culture insert with single-layer-saturated AHESS-5 cells present on the rear side of the membrane, and contact culturing between the cells separated by the membrane, i.e., the nucleus pulposus cells and AHESS-5 cells, was carried out. The AHESS-5 cell is a cell made such that a mouse marrow mesenchymal cell capable of supporting a hematopoietic stem cell generate human angiopoietin I by a genetic technique^{42, 43, 44}. Soluble angiopoietin I (500 ng/ml) was also used in the exchange of the AHESS-5. Moreover, with the purpose of investigating the effects of the AHESS-5-derived angiopoietin I, goat anti Tie2 polyclonal neutralizing antibodies or, as a control, a goat normal serum Ig (R&D system) was used at a concentration of 10µg/ml. After 7 days of mixed culturing, the NP cells were separated with TrypLE Express and provided for a colony assay and analysis by a flow cytometry method.

### [6] Colony forming test

After suspending a given number of mouse or human cells in a methocult H4230 methylcellulose medium (stem technology, no addition of growth factor), these were put in a culture dish with a diameter of 35mm and cultured for 10 days under 37°C, 5% CO₂ and 5% O₂ conditions to form a colony. The number of formed colonies was measured using an inverted phase contrast microscopy. Those composed of 10 or more cells were defined as colonies.

### [7] Flow cytometry analysis and purification of the nucleus pulposus cell

Regarding the nucleus pulposus cell, analysis of cell surface antigens was carried out using a FACS calibur flow cytometer (BD Bioscience), and cell separation was carried out using a FACS vantage cell isolating apparatus (BD Bioscience).

The human NP cells were stained using VEGF-R1 (Flt1) (R&D System, 49560), CD271 (Miltenyi, ME20.4-1.H4), CD44 (BD Bioscience, 515), CD49f (BD Bioscience, GoH3), CD56 (Beckman Coulter, N901), CD73 (BD Bioscience, AD2), CD90 (BD Bioscience, 5E10), CD105 (Beckman Coulter, 1G2), and CD166 (BD Bioscience, 3A6), which are PE-labeled mouse anti-human specific monoclonal antibodies.

After staining and washing, the NP cells were analyzed using the FACS calibur flow cytometer (BD Bioscience). Moreover, during purification of the cell, the human nucleus pulposus cells were reacted with mouse anti-human-specific disialoganglioside GD2 (GD2) monoclonal antibodies (BD Pharmingen, 14.G2a) for 30 minutes, and goat FITC-labeled anti-mouse secondary antibodies (BD Bioscience) were continuously reacted for 30 minutes at 4°C. After washing, allophycocyanin (APC)-labeled anti-human Tie2 monoclonal antibodies (R&D System, 83715) and PE-labeled anti-human CD24 monoclonal antibodies (BD Bioscience, ML5) were reacted. Regarding the mouse NP cells, an anti-GD2 monoclonal antibody, a goat biotinylated anti-mouse Tie2 polyclonal antibody and the PE-labeled anti-mouse CD24 monoclonal antibody (BD Bioscience, M1/69) were used for staining in the same manner as in humans. After washing, secondary staining was carried out using the corresponding secondary antibodies such as APC streptavidin (BD Bioscience), etc. The human or mouse nucleus pulposus cells with completed immunostaining were purified using the FACS vantage cell isolating apparatus (BD Bioscience).

### [8] Immunohistochemical staining

Immunofluorescent staining of the nucleus pulposus tissues, etc. was carried out by the method mentioned in the preceding paper 37

In the tissue immunostaining, the nucleus pulposus tissues were reacted with the following primary antibodies: an anti-human Tie2/TEK monoclonal antibody (Upstate, Ab33), a goat biotinylated anti-mouse Tie2/TEK polyclonal antibody (R&D system), an anti-human disialoganglioside GD2 mAb, (BD Biosciences, 14.G2a), an anti-human CD24 monoclonal antibody (BD Bioscience, M1/69), an anti-type II collagen monoclonal antibody (Seikagaku, II-4C11), a rabbit anti-angiopoietin I polyclonal antibody (Aclass), an anti-human cartilage proteoglycan monoclonal antibody (Chemi-con, MAB 2015), an anti-glial fibrillary acidic protein (GFAP) monoclonal antibody (Chemi-con, G-A-5), an anti-adenomatous polyposis coli (APC) monoclonal antibody (Genetics, CC-1), an anti-β tubulin monoclonal antibody (Sigma-Aldrich, 2G10), a rabbit anti-Tau polyclonal antibody (Dako), a rabbit anti-Nestin polyclonal antibody (Ab Cam), a goat anti-Nanog polyclonal antibody (R&D system), a rabbit anti-Oct4A monoclonal antibody (Cell Signaling Technology, C52G3), a rabbit anti-c-Myc polyclonal antibody (Santa Cruz), a rabbit anti-Sox2 polyclonal antibody (Chemi-con), a rabbit anti-klf4 polyclonal antibody (Santa Cruz). Next, each of the following were used as secondary antibodies (Molecular probe): a goat Alexa Fluor 488-labeled anti-mouse IgG, a goat Alexa Fluor 594-labeled anti-mouse IgG, a goat Alexa Fluor 350-labeled anti-rabbit IgG, a goat Alexa Fluor 488-labeled anti-rabbit IgG, a goat Alexa Fluor 594-labeled anti-rabbit IgG, a donkey Alexa Fluor 350-labeled anti-goat IgG. Nucleus staining with DAPI (Vector Laboratories) was also carried out on these samples at the same time. A rabbit Alexa Fluor 488-labeled anti-enhanced green fluorescent protein (EGFP) polyclonal antibody was used for reinforcing the fluorescence of the EGFP-gene-labeled transplanted cells, and an Alexa Fluor 594-labeled phalloidin (each molecular probe) was used for staining the actin filament. Moreover, a horse biotin-labeled anti-mouse antibody (Vector Laboratories) was used with an ABC complex (streptavidin /horseradish peroxidase) staining technique and DAB staining was used for counter staining with hematoxylin. The rabbit or goat IgG was used as the negative control of each immunostaining. Furthermore, bone differentiation and adipocytes differentiation of the nucleus pulposus cells was confirmed by von Kossa staining and oil red O staining. Moreover, safranin O staining or hematoxylin-eosin staining were used for observing the structure in bright field.

Image sampling and image analysis on the immunofluorescent-stained samples were carried out with LSM510 confocal laser scanning microscope (Carl Zeiss). Abode Photoshop 7.0 software (Adobe Systems) was used for image processing.

### [9] Genetic marker using a lentivirus

A high titer lentivirus encoding an EGFP reporter gene was used for genetically labeling the nucleus pulposus cells. The MOI condition was set at 10, and after carrying out infection culture for 24 hours, the nucleus pulposus cells was washed two times in the α-MEM culture medium, further cultured for 24 hours, and then provided for the experiment.

### [10] Nucleus pulposus cell transplantation

Mouse subcutaneous implant: a suspension in which the purified human nucleus pulposus cell were suspended in PBS 100 µl was subcutaneously injected and transplanted into the abdomen of the recipient NOD/SCID mouse⁴⁰. Eight weeks following transplantation, the nucleus pulposus cell cyst subcutaneously shaped in the abdomen was extirpated after putting the recipient mouse under anesthesia. The extirpated cyst was fixed with 4% paraformaldehyde (Wako Pure Chemical Industries) and used in various histological investigations.

Transplantation to the mouse caudal vertebra: the EGFP-labeled purified human nucleus pulposus cells (5.0 x 10⁵) were suspended in 50 µl PBS and subsequently injected into an insulin syringe with 27 gauge needles. Subsequently, said cell suspension was injected and transplanted into the caudal vertebra of injured NOD/SCID mouse, wherein the nucleus pulposus tissues had been absorbed with the same 27 gauge needles, at a volume of approximately 10 µl each. Said transplantations to the caudal vertebra were all carried out under a surgical microscopy. The existence or engraftment of the transplanted nucleus pulposus cells was measured by a bioluminescence imaging method (BLI method). BLI measurement was carried out using a cooled IVIS System (XenoGen), and software manufactured by XenoGen Corp. (XenoGen) was used for image analysis. The intervertebral disk of the transplanted site was extirpated 8 to 12 weeks following transplantation after putting the recipient mouse under anesthesia, and analysis was carried out. Specifically, the recipient mouse under anesthesia with isoflurane was placed in a light shielded box, and the peak EGFP fluorescence emitted from the transplanted human nucleus pulposus was detected by a CCD camera and then recorded. The EGFP fluorescence was expressed as the fluorescence quantity per second.

### [11]Apoptosis assay

Apoptosis assay of the human nucleus pulposus cells was carried out using a culture in which a goat polyclonal anti-human Tie2 neutralizing antibodies were added. a goat control Ig was used as a control. Forty-eight hours after adding the antibodies, the cells were stained with PI and FITC-labeled anti-annexin V antibodies (Beckman Coulter), and subsequently analyzed by flow cytometry using a FACS caliber.

### [12] RT-PCT

The objective nucleus pulposus cells were dissolved in a lysis buffer, and the RNA was subsequently separated using an RN aqueous-micro scale RNA isolation kit (Ambion, Inc.). The isolated mRNA was reverse transcripted using a high efficiency RNA to DNA kit (Applied Bioscience) to prepare the cDNA.

The method to amplify nine target genes by real-time PCR method was reported in the preceding paper⁴⁵. Simply put, the reverse-transcripted cDNA was amplified using a TaqMan gene expression kit (Applied Bioscience) comprising a target gene primer and a TaqMan probe (Table 1). Angiopoietin I mRNA was determined by comparative CT method (ABI prism, Applied Bioscience) with measuring 18S ribosome RNA as an internal control. Regarding the gene expression level, the expression level of each of the Tie2+GD2-, Tie2+GD2+, Tie2-GD2+ and Tie2-GD2-CD24+ cells was converted with purified nucleus pulposus Tie2-GD2-cell fractionation as the standard.

RNA extraction from the purified NP cells (Tie2+GD2+ or Tie2-GD2- cells) and bone marrow mesenchymal stromal cells (BMMSCs) was carried out by the previously mentioned method³⁷. The PCR product from 40 cycles of amplification was electrophoresed with 3% agarose gel and visualized with ethidium bromide (Sigma-Aldrich). 18S ribosomal RNA, as a control, was used as an internal standard.

**Table 1**

| TaqMan Gene Expression Assays | | | | |
|---|---|---|---|---|
| Gene Name | Gene Symble | Ref. Sequence | Assay ID | Amplicon (bp) |
| Nanog homeobox | Nanog | NM_024865 | Hs02387400_g1 | 109 |
| POU class 5 homeobox 1 | POU5F1⁺¹ | NM_203289 | Hs00742896_s1 | 65 |
| Nestin | NES | NM_006617 | Hs00707120_s1 | 81 |
| Musashi-1 | MSI1 | NM_002442 | Hs00159291_m1 | 92 |
| KIAA1217 | KIAA1217⁺² | NM_001098501 | Hs00378252_m1 | 61 |
| SRY | Sox9 | NM_000346 | Hs00165814_m1 | 102 |
| Angiopoietin-1 | ANGPT1 | NM_001146 | Hs00919202_m1 | 86 |
| Aggrecan | ACAN | NM_001135 | Hs001S3936_m1 | 91 |
| Collagen type 2 alpha1 | COL2A1 | NM_033150 | Hs1064869_m1 | 65 |
| ^{+1:}POU5F1 means OCT3/4, | ^{+2:}=KIAA1217 means SKT | | | |

### [13] Multipotency of nucleus pulposus cells

With the purpose of investigating the multipotency of human nucleus pulposus cells in vitro, induced differentiation was carried out on osteocytes, adipocytes, and chondrocytes using the method reported in the preceding paper¹. Moreover, as a method of investigating differentiation to nerve direction, Tie2+GD2+ nucleus pulposus cells at a concentration of 1.0 x 10⁴/ml were suspended in a DMEM/F12 (1:1) (GIBCO) serum-free medium with insulin (25 mg/ml), transferrin (100 mg/ml), progesterone (20 nM), sodium selenite (30 nM), putrescine-2HCL (60 nM) (all purchased from Sigma-Aldrich), genetically-modified human EGF (100 ng/ml) (Peprotech), human basic FGF (100 ng/ml) (Peprotech), and B27 (20 ng/ml) (GIBCO)² added, followed by culturing for 14 days under the conditions of 37°C, 5% CO₂ and 5% O₂. After cultivation was completed, the cells were subjected to trypsin dispersion, and 1.0 x 10³ cells were suspended in a methocult H4230 culture medium (Stemcell Technologies, growth factor free), which is a methylcellulose medium, sprayed on a 35mm culture dish, and further cultured for 7 days under the conditions of 37°C, 5% CO₂ and 5% O₂.

### [14] Statistical processing

Each outcome is shown as a mean value ± standard deviation. A student's T-test was used for the statistically significant difference. A Mann-Whitney's U-test was used in the real-time PCR method. Regarding any significant difference, a p value of 0.05 or less was determined as significant. The correlation was calculated using the Pearson correlation coefficient.

### Results

### (1) Clonogenicity of spheroid colony-forming nucleus pulposus cells

Clonogenicity and spheroid colony-forming ability are characteristics of hematopoietic stem cells, neural stem cells and ES cells ^{11,12,13}. When the NP cells collected from C57BL/6 mouse caudal vertebra intervertebral disks were cultured on methyl cellulose in order to investigate whether or not cell populations with these characteristics are present in the intervertebral disk nucleus pulposus, the derivation of several adhesive types and spherical colonies was observed. As a result of culturing for 10 days, many colonies were found to be of the adhesive type, with an adhesive type:spherical type ratio of 3.5:1 (FIG. 1a). We named these spherical colonies CFU-NP. Upon investigating many surface markers in order to specify the cell population configuring the spherical colonies by the use of flow cytometry, it was found that a small number of cell populations expressing a marker referred to as Disialoganglioside GD2 and Tie2 had a high proliferation capability. To date, a glycosylphosphatidylinositol (GPI) anchor protein CD24 has been reported as a marker of differentiated NP cells¹³. GD2 was expressed in part of the CD24-positive cells, and the derivation of spherical colonies from GD2+CD24+ cells was confirmed while derivation from GD2-CD24+ cells was not confirmed (FIG. 1b). Furthermore, a small number of NP cells, at 3-4%, were found to be tyrosine kinase receptor Tie2-positive and later differentiated into the GD2+ cells. Summarizing the results of flow cytometry, it was found that there is a hierarchy of differentiating trend such that Tie2+GD2- cells generated 10.2±2.9% (n=3) Tie2+GD2+ cells and these cells are is differentiated into Tie2-GD2-CD24+ cells (FIGS. 1 b, c and FIG. 8).

(2) Regarding Tie2+ cells, in order to search the origin of Tie2+NP cells derived from notochord-derived NP cells, we conducted research using Wnt1 and P0-Cre/Floxed-EGFP, which are both double transgenic mice with genetically marked neural crest-derived cells. These two transgenic mice are models in which EGFP is expressed in cells expressing Wnt1 or P0 genes, which may be detected with immunostaining. According to previous reports, it was determined that these genes are expressed in the notochord in addition to the neural crest-derived cells regarding only P0-cre¹⁵. When the intervertebral disks of said dual-system mice were immunostained, they were found to be positive for NP cells of P0-cre alone; therefore, it was suggested that the EGFP-positive cells are derived from notochord (FIG. 2a). Furthermore, when spherical colonies were generated on methylcellulose (FIG. 2b), most Tie2+cells and half the Tie2-cells configuring the generated colonies were found to be EGFP-positive (FIG. 2c), and it was believed that the Tie2+ cells with superior clone forming capability were derived from notochord.

### (3) Identifying the Tie2+ and GD2+ cells and Tie2/Ang-1 niche in human NP

Whether or not the trend in mouse NP cells is also observed in the human intervertebral disk was investigated using human intervertebral disk cells collected during surgery (FIG. 3a, b). The denaturing change of human intervertebral disk tissues was evaluated upon collecting, and those of Thompson grade I to III with relatively mild denaturing changes were used¹⁶. In the evaluation by immunohistostaining, very few Tie2+ cells were observed in the central part of the NP and the boundary region between the AF; meanwhile GD2+ cells were often found in cell clusters consisting of a plurality of cells and were present scattered throughout the entire NP (FIG. 3c, d). As a result of analysis by flow cytometry, the differentiating tendency of cells that may be separated by a surface marker had the same pattern as that of the mouse (FIGS. 9 to 12). Tie2 has been reported to be expressed in undifferentiated cells of nerves, blood vessels, hematopoietic organs, etc.¹⁷. The hematopoietic stem cells play a role of secreting Angiopoietin I (Ang-1) which binds with Tie2 and storing the hematopoietic stem cells in the niche in the bone marrow in a differentiation arrested state¹⁸. In order to investigate whether or not such a controlling mechanism is also present in Tie2+NP cells, Ang-1 expression in the NP tissue was immunohistochemically evaluated. Ang-1 protein was deeply expressed in almost all cells configuring the intervertebral disk tissue, in addition to being expressed in Tie2+ cells and GD2+ cells (FIGS. 3e, f). Furthermore, when secretor Ang-1 was added to NP cells cultured in serum-free medium upon methylcellulose, the number of generated spherical colonies increased; whereas, on the contrary, this declined when an antibody blocking Ang-1 were added (FIG. 3g). Moreover, the spherical colonies increased in the same manner when co-cultivation was carried out with the AHESS5 cells in which human Ang-1 was forcibly expressed (FIG. 13). Meanwhile, it was found that Tie2+ NP cells head for apoptosis in the presence of a blocking antibody against Ang-1 (FIGS. 3h, i), suggesting that a Tie2/Ang-1 signal transduction mechanism is essential in maintaining the Tie2+ NP cells, and it was clarified that the Tie2+ cells may be amplified by co-culturing using AHESS5.

### (4) Single cell analysis regarding human Tie2+GD2+ NP cells

Self-renewal from a single cell is a major stem cell trait. We proved that spherical colonies are also generated from single cell cultures of human Tie2+GD2+ NP cells (FIG. 4a) and that secondary colonies may also be generated (FIG. 4b). The major role of NP cells is the production of ECM configured from type II collagen and proteoglycan. As a result of immunostaining the spherical colonies generated by Tie2+GD2+ cells, the spherical colonies were found to be positive for Nestin, which is a marker for neural stem cells (FIG. 4d) in addition to type II collagen and proteoglycan (FIG. 4c), suggesting undifferentiation of the Tie2+GD2+ NP cells.

### (5) Tissue maintenance and re-constructing ability of Tie2+GD2+ NP cells

With the purpose of investigating whether or not Tie2+GD2+ NP cells have the ability to maintain and reconstruct the intervertebral disk tissues, first, the cells were subcutaneously injected into immunodeficient mice (FIG. 5a). Thereupon, organization construction stained with cartilage matrix was observed under the skin in 8 weeks in the group where the Tie2+GD2+ NP cells were injected (FIG. 5b). Furthermore, Tie2+GD2+ cells and Tie2-GD2-CD24+ cells genetically marked with EGFP were transplanted into the intervertebral disks of the immunodeficient mice caudal vertebra with injured intervertebral disks (FIG. 5c), followed by evaluation by bioluminescence imaging ^{19, 20}. As a result, the cells were observed only in the group with Tie2+GD2+ cells transplanted even after 12 weeks had passed: it was proved that the transplanted cells produced type II collagen and had high tissue maintenance as well as reconstructing ability (FIG. 5d).

### (6) Multipotency of human Tie2+GD2+ NP cells

The Tie2+GD2+ NP cells exhibited differentiating abilities with fat, bone, and cartilage in vitro (FIG. 6a). As a result of RT-PCR, the Tie2+GD2+ cells strongly expressed undifferentiated markers such as Nanog, Oct3/4, Nestin, Musashi-1, etc. compared to bone marrow stromal cells (FIG. 6b), and at the protein level, ES cell markers such as Oct3/4, Nanog, cMyc, Sox2, klf4, etc. were also highly expressed compared to other cell populations (FIG. 6c). Nestin and Musashi-1 are known as markers for neural stem cells ²¹, so the differentiation ability into neuro was also investigated. As a result, Tie2+GD2+ NP cells expressed a plurality of neurons and glia markers without special induction: it was found that the cells also possess a differentiating capability in the nerve direction (FIG. 6d), and the high multipotency of the Tie2+GD2+ cells was proven.

### (7) Clinical significance of human Tie2+ NP cells

In specimens from human intervertebral disk surgery, in which it is said that notochord-derived NP cells almost completely disappear when adulthood is reached ⁶, the proportion of Tie2+ cells was investigated using flow cytometry. As a result, it was found that Tie2+ cells significantly decline after a person has reached their twenties (FIG. 7a, b), and a negative correlation was indicated with the age of the donor (FIG. 7c). Furthermore, a negative correlation was indicated between the number of generated spherical colonies and age, and the usefulness of Tie2+ NP cells in pathophysiology and diagnosis on intervertebral disk degeneration was exhibited.

### Discussion

In this study, we identified the Tie2-positive population, which form spherical colonies and possess characteristics of stem cells such as self-renewal ability and multipotency, in the intervertebral disk nucleus pulposus of mice and humans for the first time by the in vitro and in vivo studies. This cell population was derived from the notochord. Moreover, it was found that Angiopoietin I was anti-apoptotic and that it plays a major role in the maintenance of said cell population, suggesting the presence of a niche environment by Angiopoietin I and the possibility of development of a treatment strategy using Angiopoietin I. It was then determined at the end that said cell populations attenuate in human intervertebral disks at a relatively early stage in the twenties, indicating that there is a close correlation between aging and degeneration of the intervertebral disks.

Forming of the spherical colonies was used as a method of separating the stem cell population with multipotency^{10-12,22}_{.} In the past, it has been reported that spherical colony forming cells in the bone marrow express the neural stem cell marker Nestin and may be differentiated into neurons, skeletal myocytes and cardiomyocytes ¹¹. The spherical colony forming cells identified in this study also expressed Nestin in addition to type II collagen and proteoglycan, which are major ECM of the intervertebral disk nucleus pulposus cells. Furthermore, Tie2 and GD2 were identified from among many stem cell-related surface markers as cell markers for purifying the spherical colony forming cells. GD2 is one of the few surface markers that may separate mesenchymal stem cells with a single marker ²³. It has been reported so far that regarding GD2-positive mesenchymal stem cells in the umbilical cord blood, ES cell markers such as Oct-4, Sox-2, Nanog, SSEA-4, etc. were expressed more than regarding GD2-negative mesenchymal stem cells ²⁴. Meanwhile, there are more reports on stem cell control via adhesion with a niche environment in hematopoietic stem cells and neural stem cells regarding Tie2 18, ²⁵. Our results added a new role to the surface markers towards nucleus pulposus cells. An irreversible differentiation tendency of nucleus pulposus cells was demonstrated upon many repetition experiments in vitro (FIG. 1c and FIGS. 9 to 11); furthermore, the hierarchy in nucleus pulposus cell differentiation was shown regarding human and mouse nucleus pulposus cells, and Ang-1 was raised as one of the niche factors regulating this (FIG. 7d).

Recently, it has been reported using Wnt1 and P0-Cre/Floxed-EGFP transgenic mice that neural-crest derived stem cells possessing multipotency and self-renewal ability are present in the bone marrow, dorsal root ganglion and whisker pad of mice ¹⁴. The intervertebral disk was not looked at in preceding research, and we investigated whether or not neural-crest derived mesenchymal stem cells may be present in the intervertebral disk ²⁶. In this study, the fact that Tie2 GD2-positive nucleus pulposus cells forming the spherical colonies were derived from the notochord is deeply related to the disappearance of notochord-derived cells in the intervertebral disk nucleus pulposus until a person reaches their twenties in addition to aging and degeneration of the intervertebral disk ³⁻⁶. There have been several reports to date indicating that notochord-derived nucleus pulposus cells apply various stimulations to other nucleus pulposus cells, making them useful for homeostasis ^{27, 28} . A decline in notochord-derived nucleus pulposus cells reduces the water content of the nucleus pulposus. Accordingly, maintaining notochord-derived nucleus pulposus cells is the ultimate goal when working out prevention and treatment strategies against intervertebral disk degeneration.

The fact that Tie2/Ang-1 signal transduction may control the fate of the cells as part of the niche environment of Tie2-positive nucleus pulposus cells is beneficial to treatment strategies. Tie2-positive cells may be proliferated and maintained by cytokine therapy. It is also important to understand the niche environment of the treatment strategy using a tissue engineering method. The Tie2/Ang-1 niche is an essential element in the regeneration of intervertebral disks.

Cell transplantation therapy is counted as a clinical trial for a therapy for inhibiting degeneration of the intervertebral disk via an animal model ²⁹⁻³². The cells include various cells marked by cartilage phenotype, for example, a chondroid cell of the intervertebral disk, articular chondrocyte, fat or bone marrow-derived stem cells, etc. ³⁰⁻³⁴. Although this is an effective technique in animal models, its effectiveness in actual clinical trials with humans still remains unknown. There have been no reports to date on research using notochord-derived nucleus pulposus cells or cells with the same characteristics thereof. From our research results, Tie2+GD2+ cells had higher repairing ability of the injured intervertebral disk than the Tie2-GD2- cells, and when considering cell transplantation, it was believed that they may become better donor cells than the cell type used in clinical trials to date. Almost all nutrients of the nucleus pulposus cell are dependent on diffusion via the upper and lower endplate cartilages; therefore, worsening of the surrounding environment with age is believed to be one factor causing decay of the notochord-derived nucleus pulposus cells. Accordingly, it is believed that cell transplantation therapy is only applicable from the early stages to the middle stages of degeneration in which the surrounding environment is relatively maintained. These various problems may be solved by further analyzing microenvironments including the notochord-derived nucleus pulposus cells and the niche thereof.

Finally, we present a differentiation cascade of the nucleus pulposus cell presumed from various results of the present study. This information will become useful in diagnoses and treatments at the clinical site. For example, the proportion of the Tie2-positive cell population that changes with age can be used as an indicator of the degree of degeneration of the intervertebral disk as well as aging. We are currently carrying out a clinical trial of cell transplantation therapy with intervertebral disk degeneration caused following surgery as the subject ²⁹, wherein the presence of such an established surface marker is very useful in evaluating the potential and quality of the cells. The present study results show new findings in the biology of intervertebral disks and are believed to be useful in the future as a new therapy against irreversibly advancing intervertebral disk disorders.

## Claims

1. An intervertebral disk nucleus pulposus cell **characterized by** being isolated from the intervertebral disk nucleus pulposus of a vertebrate, and being positive for at least one surface marker from among Tie2 and GD2.

2. The intervertebral disk nucleus pulposus cell according to claim 1, **characterized by** being a stem cell that is at least Tie2-positive for the surface marker and possesses self-renewal ability as well as multipotency capable of differentiating into adipocytes, osteocytes, chondrocytes, and neurons (hereinafter referred to as the "intervertebral disk nucleus pulposus stem cell") .

3. The intervertebral disk nucleus pulposus cell according to claim 2, **characterized by** the surface marker being GD2-negative, and being in a dormant state.

4. The intervertebral disk nucleus pulposus cell according to claim 2, **characterized by** the surface marker being GD2-positive, and being in an activated state.

5. The intervertebral disk nucleus pulposus cell according to claim 1, **characterized by** being a progenitor cell that is Tie2-negative and GD2-positive for the surface marker, and capable of differentiating into adipocytes, osteocytes, chondrocytes, or neurons (hereinafter referred to as the "intervertebral disk nucleus pulposus progenitor cell").

6. The intervertebral disk nucleus pulposus cell according to any one of claims 2 to 4, wherein the surface marker is additionally CD24-negative, CD44-positive/negative or positive, CD271-positive, and Flt1-positive.

7. The intervertebral disk nucleus pulposus cell according to claim 5, wherein the surface marker is additionally CD24-negative or positive, CD44-positive, CD271-positive/negative or negative, and Flt1-positive/negative or negative.

8. The intervertebral disk nucleus pulposus cell according to any one of claims 1 to 7, wherein the vertebrate is a mammal.

9. The intervertebral disk nucleus pulposus cell according to claim 8, wherein the mammal is a human or a mouse.

10. A cultivation method for an intervertebral disk nucleus pulposus cell **characterized by** comprising: isolating a cell positive for at least one surface marker from among Tie2 and GD2 from a nucleus pulposus cell population collected from the intervertebral disk nucleus pulposus of a vertebrate or a cell population obtained by cultivating the same.

11. The cultivation method for an intervertebral disk nucleus pulposus cell according to claim 10, **characterized by** said isolated cell being the intervertebral disk nucleus pulposus stem cell which is at least Tie2-positive for the surface marker.

12. The cultivation method for an intervertebral disk nucleus pulposus cell according to claim 10, **characterized by** said isolated cell being the intervertebral disk nucleus pulposus progenitor cell which is Tie2-negative and GD2-negative for the surface marker.

13. The cultivation method for an intervertebral disk nucleus pulposus cell according to claim 11, comprising cultivating an intervertebral disk nucleus pulposus stem cell in the presence of angiopoietin I (Ang-1).

14. A cultivation method for adipocytes, osteocytes, chondrocytes, or neurons **characterized by** comprising:
cultivating the intervertebral disk nucleus pulposus cell according to any one of claims 1 to 9, or the intervertebral disk nucleus pulposus cell obtained through the cultivation method according to any one of claims 10 to 13, under differentiation-inducing conditions.

15. A cell composition **characterized by** comprising the intervertebral disk nucleus pulposus cell according to any one of claims 1 to 9, or the intervertebral disk nucleus pulposus cell obtained through the cultivation method according to any one of claims 10 to 13.

16. The cell composition according to claim 15, a use thereof being for the treatment or prevention of intervertebral disc disorder.

17. A method for treating or preventing intervertebral disc disorder in vertebrates (excluding humans), the method comprising transplanting the intervertebral disk nucleus pulposus cell according to any of claims 1 to 9, the intervertebral disk nucleus pulposus cell obtained through the cultivation method according to any one of claims 10 to 13, or the cell composition according to claim 15 or 16 to an intervertebral disk.

18. A method for treating or preventing intervertebral disc disorder in vertebrates (excluding humans), comprising administering Ang-1 to the intervertebral disk nucleus pulposus stem cell in the intervertebral disk.

19. A method for obtaining an indicator related to the state of an intervertebral disk, the method **characterized by** comprising:
measuring the proportion of intervertebral disk nucleus pulposus stem cells in a nucleus pulposus cell population sampled from the intervertebral disk nucleus pulposus of a vertebrate.
